(19) Europäisches Patentamt
European Patent Office
Office européen des brevets

(11) Veröffentlichungsnummer: **0 088 276**
**B1**

(12) # EUROPÄISCHE PATENTSCHRIFT

(45) Veröffentlichungstag der Patentschrift: **30.04.86**

(21) Anmeldenummer: **83101626.6**

(22) Anmeldetag: **21.02.83**

(51) Int. Cl.⁴: **C 07 D 211/90**, C 07 D 309/32, C 07 D 401/04, C 07 D 403/04, C 07 D 413/04, C 07 D 417/04, A 61 K 31/445

(54) **Neue Verbindungen, Verfahren zu ihrer Herstellung sowie ihre Verwendung als Arzneimittel.**

(30) Priorität: **10.03.82 DE 3208628**

(43) Veröffentlichungstag der Anmeldung: **14.09.83 Patentblatt 83/37**

(45) Bekanntmachung des Hinweises auf die Patenterteilung: **30.04.86 Patentblatt 86/18**

(84) Benannte Vertragsstaaten: **AT BE CH DE FR GB IT LI LU NL SE**

(56) Entgegenhaltungen:
EP-A-0 000 150
EP-A-0 012 180
EP-A-0 042 089
EP-A-0 052 300
DE-A-2 117 571
DE-A-2 117 573
DE-A-2 235 406
DE-A-2 650 013
FR-A-2 376 137
GB-A-2 049 671

**CHEMICAL ABSTRACTS, Band 83, 1975, Seite 493, Nr. 28058n, Columbus, Ohio, USA A. SAUSINS et al.: "4-Substituted 2,6-dimethyl-3,5-di-beta-alkoxyet hoxy carbonyl-1,4-dihydropyridines"**

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Geilertweg 33, D-5600 Wuppertal 1 (DE)**
Erfinder: **Meyer, Horst, Dr., Henselweg 11, D-5600 Wuppertal 1 (DE)**
Erfinder: **Knorr, Andreas, Dr., Pahlkestrasse 15, D-5600 Wuppertal 1 (DE)**

(56) Entgegenhaltungen: (Fortsetzung)
**CHEMICAL ABSTRACTS, Band 98, 1983, Seite 622, Nr. 125898d, Columbus, Ohio, USA**

***Die Akte enthält technische Angaben, die nach dem Eingang der Anmeldung eingereicht wurden und die nicht in dieser Patentschrift enthalten sind.***

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 088 276 B1

LIBER, STOCKHOLM 1986

**Beschreibung**

Die vorliegende Erfindung betrifft neue Verbindungen mit langanhaltender Kreislaufwirkung, mehrere Verfahren zu ihrer Herstellung sowie ihre Verwendung als kreislaufbeeinflussende Arzneimittel, insbesondere als blutdrucksenkende Mittel.

Es ist bereits bekannt geworden, daß man 1,4-Dihydro-2,6-dimethyl-4-phenyl-pyridin-3,5-dicarbonsäurediethylester erhält, wenn man 2-Benzylidenacetessigsäureethyl-ester mit β-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1,4-Dihydropyri-dine interessante pharmakologische Eigenschaften aufweisen (F. Bossert, W. Vater, Naturwissenschaften 58, 578 (1971)).

Weiterhin ist aus den deutschen Offenlegungsschriften 2 117 571 und 2 117 573 bekannt geworden, daß ähnliche Verbindungen als Coronarmittel und als blutdrucksenkende Mittel verwendet werden können.

Bei Kenntnis dieses Standes der Technik konnte jedoch nicht erwartet werden, daß die neuen erfindungsgemäßen Verbindungen eine so überraschend langanhaltende blutdrucksenkende Wirkung besitzen.

Die Erfindung betrifft neue Verbindungen der allgemeinen Formel (I)

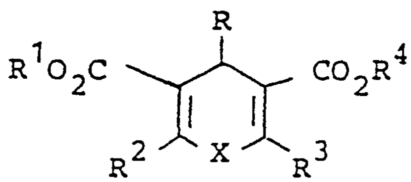

(I)

in welcher

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxymethylen, Halogen, Trifluormethyl, Trifluormethoxy, Difluormethoxy, Tetrafluorethoxy, Nitro, Cyano, Azido oder $SO_m$-Alkyl, worin m eine Zahl von 0 bis 2 bedeutet und Alkyl vorzugsweise 1 bis 4 Kohlenstoffatome enthält, substituiert sein können,

$R^1$ für einen geradkettigen, verzweigten oder cyclischen, gesättigten oder ungesättigten Kohlenwasserstoffrest mit bis zu 20 Kohlenstoffatomen steht, der gegebenenfalls durch 1 Sauerstoffatom in der Kette unterbrochen ist und/oder der gegebenenfalls substituiert ist durch Halogen, Cyano, Hydroxy, Acetoxy, Phenyl, Phenoxy oder eine Aminogruppe, die ihrerseits durch 2 gleiche oder verschiedene Substituenten aus der Gruppe Alkyl mit 1 bis 4 Kohlenstoffatomen, Phenyl oder Benzyl substituiert ist,

$R^4$ für einen geradkettigen, verzweigten oder cyclischen, gesättigten oder ungesättigten aliphatischen Kohlenwasserstoffrest mit 7 bis 14 Kohlenstoffatomen steht, der gegebenenfalls durch ein Sauerstoffatom in der Kette unterbrochen ist und/ oder der gegebenenfalls durch Halogen, Hydroxy oder Acetoxy substituiert ist,

$R^2$ und $R^3$ gleich oder verschieden sind und jeweils für Wasserstoff, einen geradkettigen oder verzweigten Alkylrest mit bis zu 4 Kohlenstoffatomen, einen Phenylrest oder einen Benzylrest stehen, oder einer der Substituenten $R^2$ oder $R^3$ für eine Hydroxymethyl-, Acetoxymethyl- oder Aminogruppe steht, wobei der andere Substituent die oben angegebene Bedeutung besitzt, und

X für N-$R^5$ steht, wobei $R^5$ für Wasserstoff, einen geradkettigen oder verzweigten, gegebenenfalls durch ein Sauerstoffatom unterbrochenen Alkylrest mit bis zu 4 Kohlenstoffatomen, einen Morpholinoethylrest, einen Phenylrest oder einen Benzylrest, steht,

oder

für ein Sauerstoffatom steht, wobei dann einer der Substituenten $R^2$ oder $R^3$ immer eine Aminogruppe bedeutet. sowie ihre pharmazeutisch unbedenklichen Säureadditionssalze.

Die Herstellung der erfindungsgemäßen Verbindungen kann nach mehreren Verfahren erfolgen, die sich im wesentlichen an die bekannte Hantzsche Pyridinsynthese anlehnen. Je nach Art der unterschiedlichen Substituentenbedeutungen seien im folgenden die Untervarianten dieses Syntheseweges beschrieben. Die Varianten A) bis G) beziehen sich jeweils auf die Herstellung von Dihydropyridinen. Die Variante H betrifft einen nachträglichen Substituentenaustausch in 2-Position des Dihydropyridinringes und die Variante J betrifft die Herstellung von 4H-Pyranen.

Für den Fall, daß X in der allgemeinen Formel (I) für N-$R^5$ steht, wobei $R^5$ die oben angegebene Bedeutung hat und

$R^{2'}$ und $R^{3'}$ jeweils für Wasserstoff, einen geradkettigen oder verzweigten Alkylrest, einen Arylrest, einen Aralkylrest oder für die Acetoxymethylgruppe steht, erfolgt die Herstellung der erfindungsgemäßen Verbindungen vorzugsweise dadurch, daß man

A)

Yliden-β-ketoester der allgemeinen Formel (II)

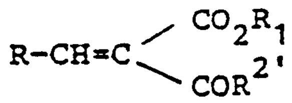 (II)

in welcher
R, $R^1$ und $R^{2'}$ die oben angegebene Bedeutung haben,
mit Enaminocarbonsäureestern der allgemeinen Formel (III)

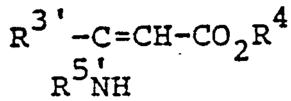 (III)

in welcher
$R^{3'}$, $R^5$ und $R^4$ die oben angegebene Bedeutung haben,
gegebenenfalls in Gegenwart inerter organischer Lösungsmittel umsetzt,
oder
B)
Yliden-β-ketoester der allgemeinen Formel (II)

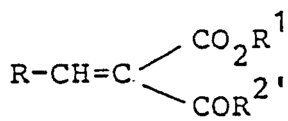 (II)

in welcher
R, $R^1$ und $R^{2'}$ die oben angegebene Bedeutung haben
mit Aminen der allgemeinen Formel (IV) und β-Ketocarbonsäureestern der allgemeinen Formel (V)

$$R^5-NH_2 \quad (IV) \qquad\qquad R^{3'}-CO-CH_2-CO_2R^4 \quad (V)$$

in welchen
$R^5$, $R^{3'}$ und $R^4$ die oben angegebene Bedeutung haben,
gegebenenfalls in Gegenwart inerter organischer Lösungsmittel umsetzt,
oder
C)
Yliden-β-ketoester der allgemeinen Formel (VI)

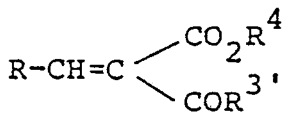

(VI)

in welcher
$R$, $R^{3'}$ und $R^4$ die oben angegebene Bedeutung haben,
mit Enaminocarbonsäureestern der allgemeinen Formel (VII)

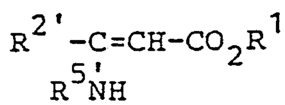

(VII)

in welcher
$R^{2'}$, $R^5$ und $R^1$ die oben angegebene Bedeutung haben, gegebenenfalls in Gegenwart inerter organischer Lösungsmittel zur Reaktion bringt,
oder
D)
Yliden-β-ketoester der allgemeinen Formel (VI)

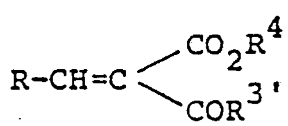

(VI)

in welcher
$R$, $R^{3'}$ und $R^4$ die oben angegebene Bedeutung haben,
mit Aminen der allgemeinen Formel (IV)
$R^5$-$NH_2$ (IV)
in welcher
$R^5$ die oben angegebene Bedeutung hat,
und β-Ketocarbonsäureestern der allgemeinen Formel (VIII)
$R^{2'}$-CO-$CH_2$-$CO_2R^1$ (VIII)
in welcher
$R^{2'}$ und $R^1$ die oben angegebene Bedeutung haben,
gegebenenfalls in Gegenwart inerter organischer Lösungsmittel umsetzt
oder
E)
Aldehyde der allgemeinen Formel (IX)

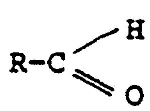

(IX)

in welcher
R die oben angegebene Bedeutung hat
mit Enaminocarbonsäureestern der allgemeinen Formel (III)

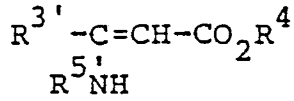

(III)

in welcher
$R^{3'}$, $R^4$ und $R^5$ die oben angegebene Bedeutung haben,
und mit β-Ketocarbonsäureestern der allgemeinen Formel (VIII)
$R^{2'}$-CO-$CH_2$-$CO_2R^1$ (VIII)
in welcher
$R^1$ und $R^{2'}$ die oben angegebene Bedeutung haben,
gegebenenfalls in Gegenwart inerter organischer Lösungsmittel umsetzt,
oder
F)
Aldehyde der allgemeinen Formel (IX)

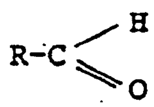

(IX)

in welcher
R die oben angegebene Bedeutung hat,
mit Enaminocarbonsäureestern der allgemeinen Formel (VII)

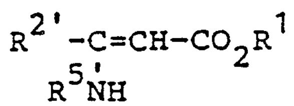

(VII)

in welcher
$R^1$, $R^{2'}$ und $R^5$ die oben angegebene Bedeutung haben
und mit β-Ketocarbonsäureestern der allgemeinen Formel (V)
$R^{3'}$-CO-$CH_2$-$CO_2R^4$ (V)
in welcher
$R^{3'}$ und $R^4$ die oben angegebene Bedeutung haben,
gegebenenfalls in Gegenwart inerter organischer Lösungsmittel zur Reaktion bringt,
oder
G)
für den Fall, daß X in Formel (I) NH bedeutet und $R^2$ oder $R^3$ eine Aminogruppe darstellen, werden die stellt, indem man
1.Yliden-β-ketoester der allgemeinen Formel (II)

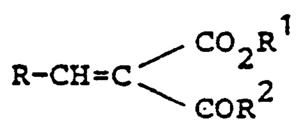

(II)

in welcher
R, $R^1$ und $R^2$ die oben angegebene Bedeutung haben, aber $R^2$ nicht für eine Amino- oder Hydroxymethylgruppe steht,
mit Amidinoessigsäureestern der allgemeinen Formel (X)

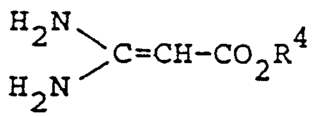

(X)

in welcher
$R^4$ die oben angegebene Bedeutung hat,
gegebenenfalls in Gegenwart inerter organischer Lösungsmittel umsetzt
oder
2. Yliden-β-ketoester der allgemeinen Formel (VI)

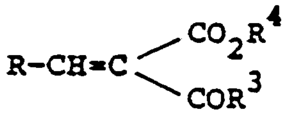

(VI)

in welcher
R, $R^3$ und $R^4$ die oben angegebene Bedeutung haben, aber $R^3$ nicht für eine Amino- oder Hydroxymethylgruppe steht,
mit Amidinoessigsäureestern der allgemeinen Formel (XI)

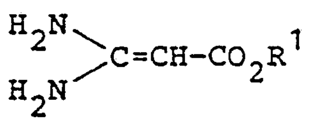

(XI)

in welcher
$R^1$ die oben angegebene Bedeutung hat,
gegebenenfalls in Gegenwart inerter organischer Lösungsmittel zur Reaktion bringt
oder
H)
für den Fall, daß X in der allgemeinen Formel (I) für N-$R^5$ steht, wobei $R^5$ die oben angegebene Bedeutung hat, und
$R^2$ oder $R^3$ für die Hydroxymethylgruppe stehen, werden die erfindungsgemäßen Verbindungen vorzugsweise hergestellt, indem man die entsprechenden erfindungsgemäßen Verbindungen, bei denen $R^2$ oder $R^3$ Acetoxymethyl bedeuten, den Bedingungen einer sauren oder basischen Hydrolyse unterwirft, wobei die Acetoxymethylgruppe in eine Hydroxymethylgruppe überführt wird,
oder
I)
für den Fall, daß X in der allgemeinen Formel (I) ein Sauerstoffatom darstellt und $R^2$ oder $R^3$ für eine Aminogruppe steht, werden die erfindungsgemäßen Verbindungen vorzugsweise hergestellt, indem man
1. Yliden-β-ketoester der allgemeinen Formel (II)

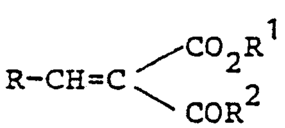

(II)

in welcher
R, $R^1$ und $R^2$ die oben angegebene Bedeutung haben aber $R^2$ nicht für die Amino- oder Hydroxymethylgruppe

steht,

mit Cyanessigsäureestern der allgemeinen Formel (XII)

$N{\equiv}C\text{-}CH_2\text{-}CO_2R^4$ (XII)

in welcher

$R^4$ die oben angegebene Bedeutung hat,

gegebenenfalls in Gegenwart organischer Lösungsmittel umsetzt,

oder

2. Yliden-β-ketoester der allgemeinen Formel (VI)

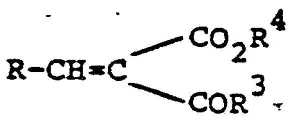

(VI)

in welcher

R, $R^4$ und $R^3$ die oben angegebene Bedeutung haben, aber $R^3$ nicht für die Amino- oder Hydroxymethylgruppe steht,

mit Cyanessigsäureestern der allgemeinen Formel (XIII)

$N{\equiv}C\text{-}CH_2\text{-}CO_2R^1$ (XIII)

in welcher

$R^1$ die oben angegebene Bedeutung hat,

gegebenenfalls in Gegenwart organischer Lösungsmittel zur Reaktion bringt.

Die erfindungsgemäßen Verbindungen besitzen wertvolle pharmakologische Eigenschaften. Insbesondere aufgrund ihrer unerwarteten langanhaltenden kreislaufbeeinflussenden Wirkung können sie als antihypertensive Mittel, als Vasodilatatoren, als Cerebraltherapeutika sowie als Coronartherapeutika Verwendung finden und sind somit als Bereicherung der Pharmazie anzusehen.

Von besonderem Interesse ist die langanhaltende Blutdruckwirkung.

Je nach Art der verwendeten Ausgangsstoffe können die Synthese-Varianten für die erfindungsgemäßen Verbindungen durch folgende Formelschemata wiedergegeben werden:

A)

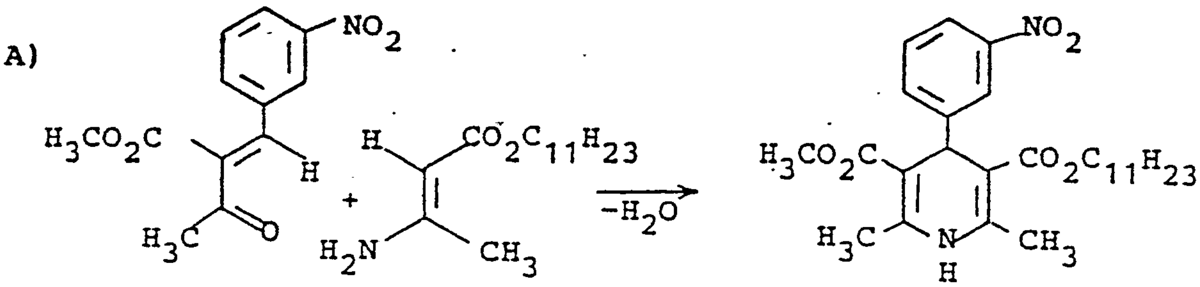


B)

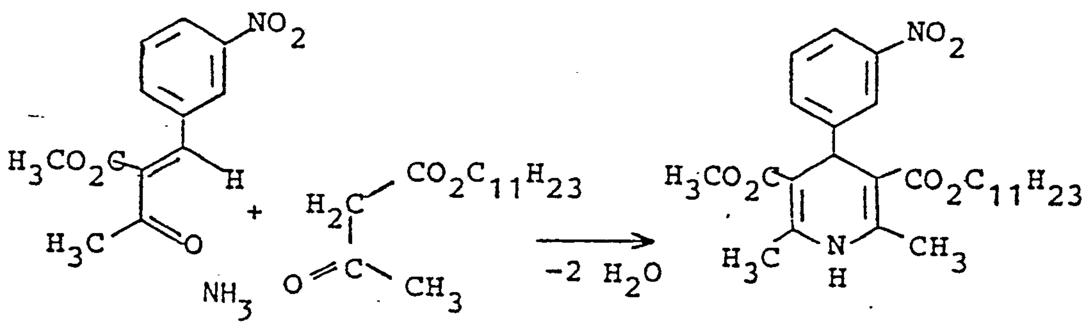


C)

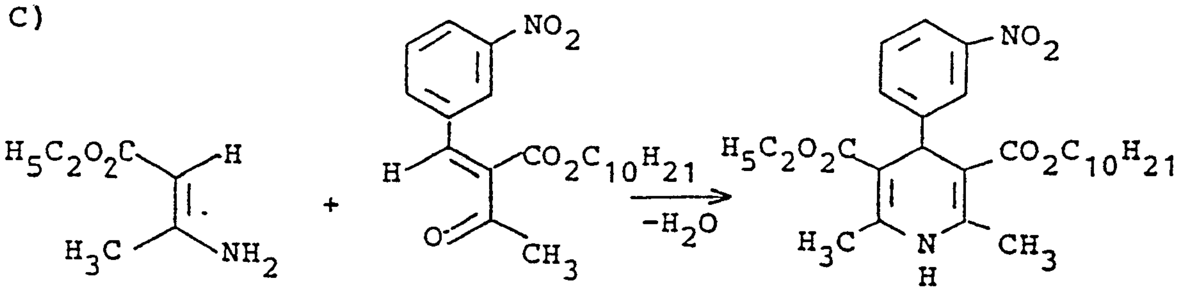

D)

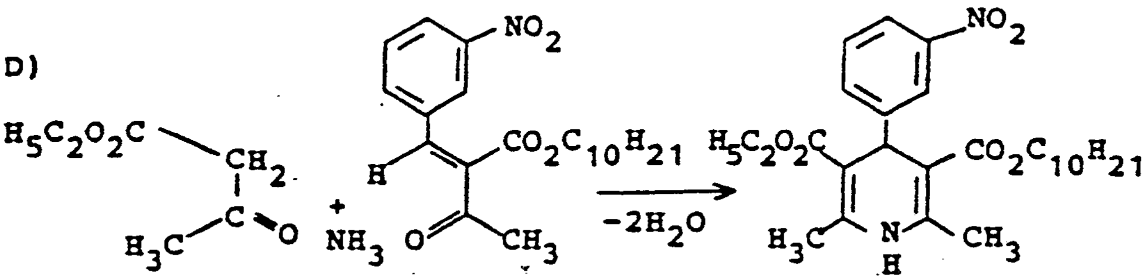


E)

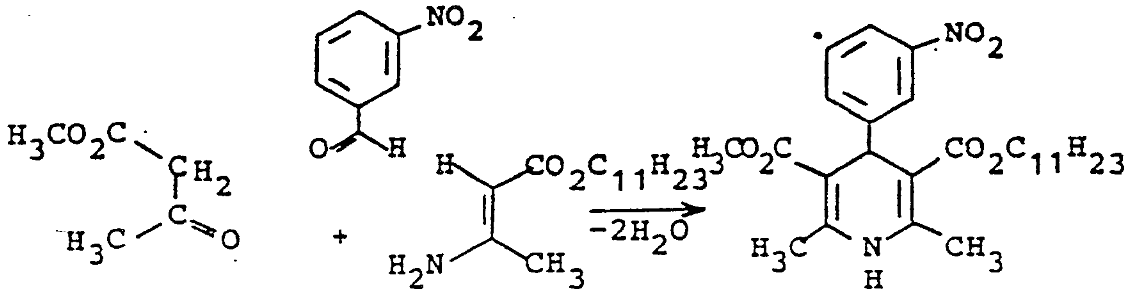


F)

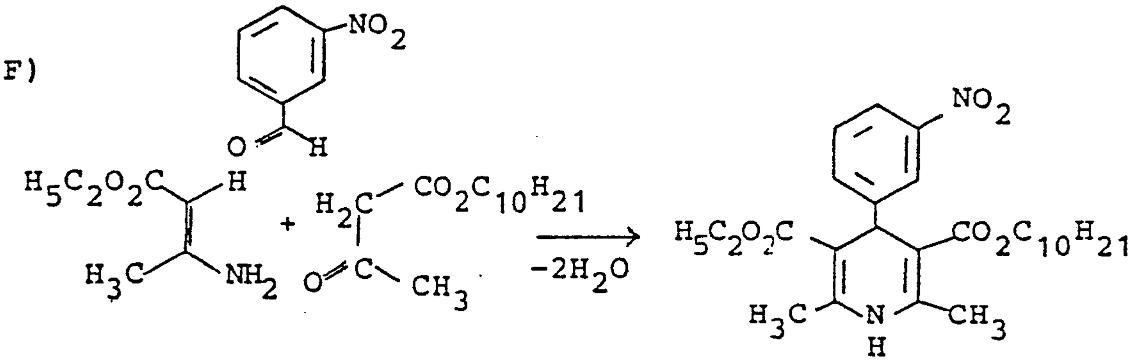


G)

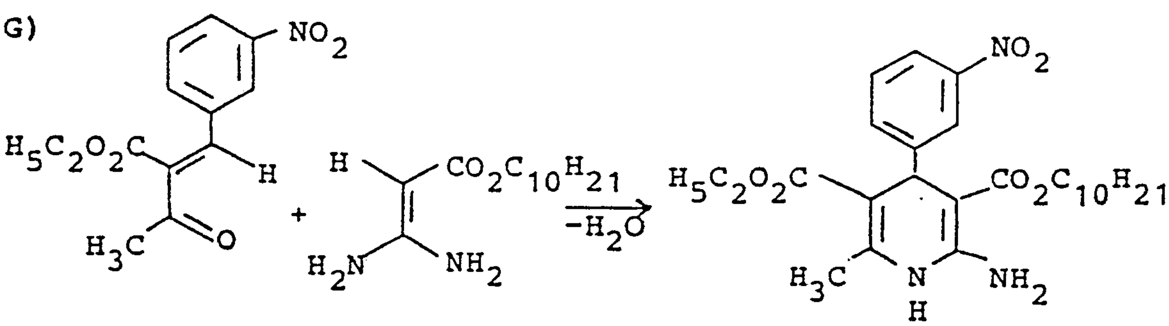

H)

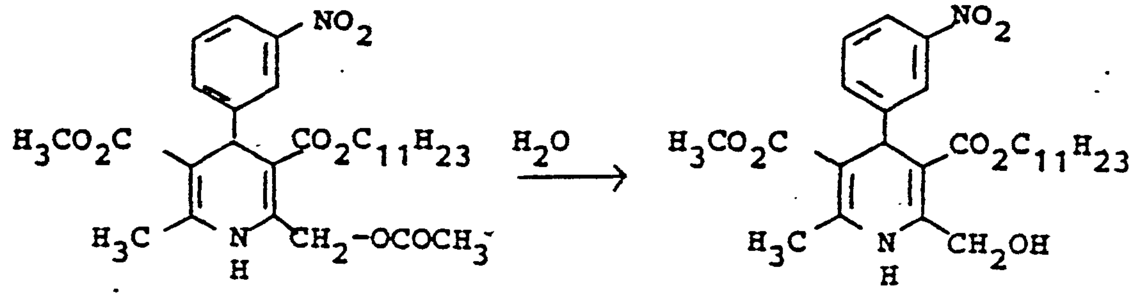

I)

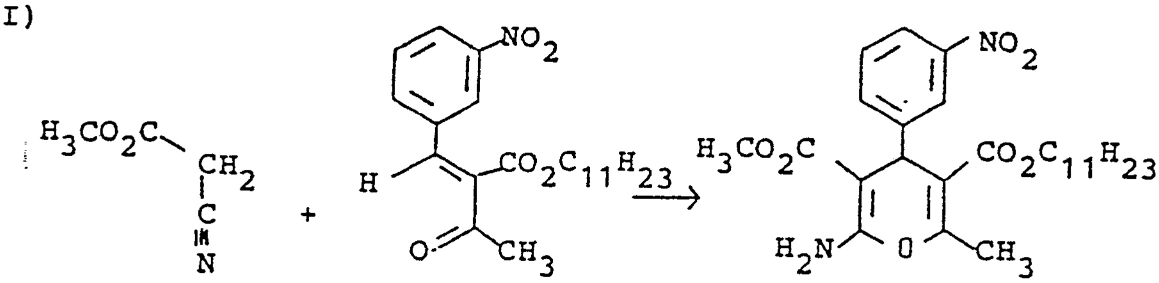

Von besonderem Interesse sind die erfindungsgemäßen Verbindungen der allgemeinen Formel (I)

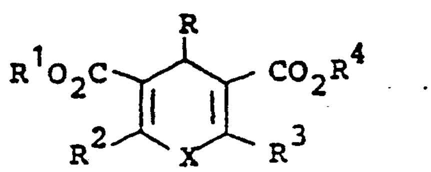

(I)

Vorzugsweise seien Verbindungen der allgemeinen Formel (I) genannt
in welcher

R für Phenyl, Pyridyl, Thienyl oder Furyl steht, wobei der Phenylring gegebenenfalls durch ein oder zwei gleiche oder verschiedene Substituenten aus der Gruppe Phenyl, Alkyl mit 1 bis 4 Kohlenstoffatomen, Alkoxy mit 1 bis 2 Kohlenstoffatomen, Fluor, Chlor, Brom, Trifluormethyl, Trifluormethoxy, Difluormethoxy, Nitro, Cyano, Azido oder Alkylmercapto mit 1 bis 4 Kohlenstoffatomen im Alkylrest substituiert ist,

$R^1$ für einen geradkettigen verzweigten oder cyclischen, gesättigten oder ungesättigten Kohlenwasserstoffrest mit bis zu 14 Kohlenstoffatomen steht der gegebenenfalls durch ein Sauerstoffatom unterbrochen ist und/oder der gegebenenfalls substituiert ist durch Fluor, Chlor, Cyano, Hydroxy oder Amino,

$R^4$ für einen geradkettigen, verzweigten oder cyclischen, gesättigten oder ungesättigten aliphatischen Kohlenwasserstoffrest mit 7 bis 12 Kohlenstoffatomen steht, der gegebenenfalls durch ein Sauerstoffatom in der Kette unterbrochen ist und/oder der gegebenenfalls durch Fluor, Chlor, Hydroxy oder Acetoxy substituiert ist,

$R^2$ und $R^3$ gleich oder verschieden sind und jeweils für Wasserstoff, Alkyl mit 1 bis 2 Kohlenstoffatomen, Phenyl oder Benzyl stehen

oder

einer der Substituenten $R^2$ oder $R^3$ für eine Hydroxymethyl-, Acetoxymethyl- oder Aminogruppe steht, wobei der andere Substituent die vorher angegebene Bedeutung besitzt,

X für $N-R^5$ steht, wobei $R^5$ für Wasserstoff, einen Alkylrest mit bis zu 4 Kohlenstoffatomen der gegebenenfalls durch ein Sauerstoffatom unterbrochen ist, einen Morpholinoalkylrest mit 1 bis 4 Kohlenstoffatomen im Alkylteil, einen Phenylrest oder einen Benzylrest steht,

oder

für ein Sauerstoffatom steht wobei dann einer der Substituenten $R^2$ oder $R^3$ immer eine Aminogruppe bedeutet.

Ganz besonders hervorgehoben seien Verbindungen der allgemeinen Formel (I)

in welcher

R für einen Phenylrest steht, der ein oder zwei gleiche oder verschiedene Substituenten aus der Gruppe Chlor, Trifluormethyl, Nitro oder Cyano enthält

oder für einen Benzoxadiazolylrest steht,

$R^1$ einen geradkettigen oder verzweigten Kohlenwasserstoffrest mit bis zu 7 Kohlenstoffatomen darstellt, der gegebenenfalls durch ein Sauerstoffatom in der Kette unterbrochen ist und/oder der gegebenenfalls durch Fluor oder Chlor oder eine Aminogruppe substituiert ist, die ihrerseits durch zwei Methylgruppen oder durch eine Methyl- und eine Benzylgruppe substituiert sein kann,

$R^4$ einen geradkettigen, verzweigten oder cyclischen, gesättigten oder ungesättigten aliphatischen Kohlenwasserstoffrest mit sieben bis zwölf Kohlenstoffatomen bedeutet, der gegebenenfalls durch ein Sauerstoffatom in der Kette unterbrochen ist oder der gegebenenfalls durch Fluor, Chlor oder Hydroxy substituiert ist,

$R^2$ und $R^3$ jeweils für Wasserstoff oder einen Alkylrest mit 1 bis 2 Kohlenstoffatomen stehen,

oder einer der Substituente $R^2$ oder $R^3$ für die Aminogruppe steht wobei der andere Substituent die oben angegebene Bedeutung hat, und

X für $N-R^5$ steht, wobei $R^5$ ein Wasserstoffatom oder einen Alkylrest mit bis zu 3 Rohlenstoffatomen darstellt,

oder

für ein Sauerstoffatom steht, wobei einer der Substituenten $R^2$ oder $R^3$ immer eine Aminogruppe darstellt.

**Verfahrensvariante A**

Gemäß Verfahren A wird ein Yliden-β-ketoester der allgemeinen Formel II

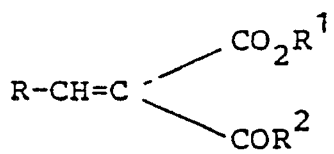

(II)

mit einem Enaminocarbonsäureester der allgemeinen Formel III

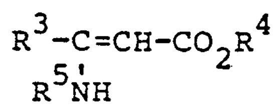

(III)

zur Reaktion gebracht.

Die als Ausgangsstoffe verwendeten Yliden-β-ketoester der allgemeinen Formel (II) sind literaturbekannt oder können nach literaturbekannten Methoden hergestellt werden (vgl. z.B. G. Jones "The Knoevenagel Condensation" in Org. Reactions, Vol. XV, 204 ff (1969)).

Als Beispiele seien genannt:
Benzylidenformylessigsäuremethylester,
Benzylidenacetessigsäuremethylester,
2-Nitrobenzylidenacetessigsäureethylester,
3-Nitrobenzylidenacetessigsäure-n-butylester,
3-Nitrobenzylidenacetessigsäuredecylester,
3-Nitrobenzylidenacetessigsäuredodecylester,
3-Nitrobenzylidenacetessigsäuretetradecylester,
2,3-Dichlorbenzylidenacetessigsäureethylester,
2,3-Dichlorbenzylidenacetessiqsäuredecylester,
2-Chlorbenzylidenacetessigsäurehexadecylester,
3-Chlorbenzylidenacetessigsäure-(2-propoxyethyl)-ester,
3-Cyanobenzylidenacetessigsäure-(2-chlorethyl)-ester,
2-Chlorbenzylidenacetessigsäure-(2.2.2-trifluorethyl)-ester,
2-Trifluormethylbenzylidenacetessigsäure-(2-cyano-ethyl)-ester,

2-Difluormethoxybenzylidenacetessigsäure-(2-acetoxy-ethyl)-ester,
3-Methylsulfonylbenzylidenacetessigsäure-(2-phenoxyethyl)-ester,
3-Nitrobenzylidenacetessigsäure-(2-N-benzyl-N-methyl-aminoethyl)-ester,
3-Nitrobenzylidenpropionylessigäureisopropylester,
2-Nitrobenzylidenacetessigsöurecyklopentylester,
2-Nitrobenzylidenacetessigsäureisobutylester,
3-Nitrobenzylidenacetessigsäureneopentylester,
3- nitrobenzylidenacetessigsäureallylester,
α-Acetyl-β-(pyridyl-3)-acrylsäureethylester,
α-Acetyl-β-(pyridyl-3)-acrylsäureoctylester,
α-Acetyl-β-(pyridyl-3)-acrylsäuredecylester,
α-Acetyl-β-(pyridyl-3)-acrylsäuretetradecylester,
α-Acetyl-β-(benzoxadiazolyl-4)-acrylsäureethylester.

Die als Ausgangsstoffe verwendeten Enaminocarbonsäureester der allgemeinen Formel (III) sind literaturbekannt oder können nach literaturbekannten Methoden hergestellt werden (vgl. A. C. Cope, J. Amer. chem. Soc. 67, 1017 (1945)).

Als Beispiele seien genannt:
β-Aminocrotonsäureheptylester,
β-Aminocrotonsäureoctylester,
β-Aminocrotonsäurenonylester,
β-Aminocrotonsäuredecylester,
β-Aminocrotonsäureundecylester,
β-Aminocrotonsäuredodecylester,
β-Aminocrotonsäuretetradecylester,
β-Aminocrotonsäure-(2-pentyloxyethyl)-ester,
β-Aminocrotonsäure-(2-hexyloxyethyl)-ester,
β-Aminocrotonsäureheptylester,
β-Aminocrotonsäureoctenylester,
β-Aminocrotonsäure-( ω -chlorheptyl)-ester,
β-Aminocrotonsäure-( ω -chlordecyl)-ester,
β-Aminocrotonsäure-( ω-hydroxydecyl)-ester,
β-Aminocrotonsäure-( ω-acetoxydecyl)-ester,
β-Aminocrotonsäure-( ω-hydroxyundecyl)-ester,
β-Aminocrotonsäure-( ω-acetoxyundecyl)-ester,
β-Methylaminocrotonsäuredecylester,
β-Benzylaminocrotonsäuredecylester,
β-Amino-β-ethyl-acrylsäuredecylester,
β-Amino-β-propyl-acrylsäuredecylester,
β-Amino-β-benzyl-acrylsäuredecylester,
β-Amino-β-acetoxymethyl-acrylsäuredecylester,
β-Amino-β-acetoxymethylacrylsäureundecylester.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise zwischen 20 und 100°C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des Yliden-β-ketoesters der Formel (II) mit einem Mol Enaminocarbonsäureester der Formel (III) in einem geeigneten Lösungsmittel zur Reaktion gebracht. Die Isolierung und Reinigung der erfindungs/gemäßen Substanzen erfolgt vorzugsweise derart, daß man das Lösungsmittel im Vakuum abdestilliert und den gegebenenfalls erst nach Eiskühlung kristallin erhaltenen Rückstand aus einem geeigneten Lösungsmittel umkristallisiert.

**Verfahrensvariante B**

Gemäß Verfahren B wird ein Yliden-β-ketoester der allgemeinen Formel II

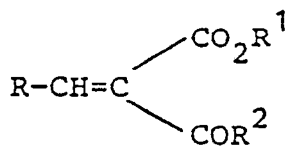

(II)

mit einem Amin der allgemeinen Formel (IV) und einem β-Ketocarbonsäureester der allgemeinen

$R^5\text{-}NH_2$ $R^3\text{-}CO\text{-}CH_2\text{-}CO_2R^4$

(IV) (V)

Formel (V) zur Reaktion gebracht.

Beispiele der als Ausgangssubstanzen verwendeten Yliden-β-ketoester der allgemeinen Formel II sind bereits unter Verfahrensvariante A aufgeführt.

Die erfindungsgemäß verwendbaren Amine der Formel (IV) sind bereits bekannt.

Als Beispiele seien genannt:

Ammoniak, Methylamin, n-Propylamin, Isopropylamin, Butylamin, β-Methoxyethylamin, Benzylamin, 2-(N-Morpholinyl)-ethylamin.

Die als Ausgangsstoffe verwendeten β-Ketocarbonsäureester der allgemeinen Formel (V) sind literaturbekannt oder können nach literaturbekannten Methoden hergestellt werden (z.B. D. Borrmann, "Umsetzung von Diketen mit Alkoholen, Phenolen und Mercaptanen", in Houben-Weyl, Methoden der Organischen Chemie, Vol. V11/4, 230 ff (1968); Y. Oikawa, K. Sugano und 0. Yonemitsu, J. Org. Chem. 43, 2087 (1978)).

Als Beispiele seien genannt:

Acetessigsäureheptylester,
Acetessigsäurenonylester,
Acetessigsäuredecylester,
Acetessigsäureundecylester,
Acetessigsäuretetradecylester,
Acetessigsäure-(2-pentyloxyethyl)-ester,
Acetessigsäureheptenylester,
Acetessigsäure-(ω-chlorheptyl)-ester,
Acetessigsäure-(ω-hydroxydecyl)ester,
Acetessigsäure-(ω-acetoxyundecyl)-ester,
Propionylessigsäuredecylester,
Butyrylessigsäuredecylester.

Als verdünnungsmittel kommen alle inerten organischen Lösungsmittel im Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgefürt werden. Im allgemeinen arbeitet man unter Normaldruck.

Bei der Durchfürung des erfindungsgemäßen Verfahrens werden die an der Reaktion beteiligten Stoffe der Formeln (II), (IV) und (V) jeweils in molaren Mengen eingesetzt. Das verwendete Amin wird zweckmäßigerweise im Überschuß von 1 bis 2 Mol zugegeben. Die erfindungs-gemäßen Verbindungen können leicht durch Umkristallisation aus einem geeigneten Lösungsmittl gereinigt werden.

**Verfahrensvariante C**

Gemäß Verfahren C wird ein Yliden-β-ketoester der allgemeinen Formel (VI)

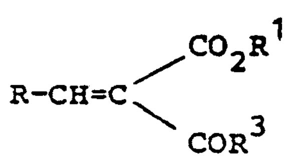

(VI)

mit einem Enaminocarbonsäureester der allgemeinen Formel (VII)

$$R^2-\underset{\underset{R^5NH}{|}}{C}=CH-CO_2R^1 \qquad (VII)$$

zur Reaktion gebracht.

Die als Ausgangsstoffe verwendeten Yliden-β-ketoester der Formel (VI) sind literaturbekannt oder können nach literaturbekannten Methoden hergestellt werden (vgl. z.B. G. Jones "The Knoevenagel Condensation" in Org. Reaktions, Vol. XV, 204 ff (1967)).

Als Beispiele seien genannt:
Benzylidenacetessigsäureheptylester,
3'-Nitrobenzylidenacetessigsäureheptylester,)
2-Chlorbenzylidenacetessigsäureoctylester,
2-Cyanobenzylidenacetessigsäurenonylester,
3-Nitrobenzylidenacetessigsäuredecylester,
2-Trifluormethylbenzylidenacetessigsäuredecylester,
2,3-Dichlorbenzylidenacetessigsäuredecylester,
3-Nitrobenzylidenacetessigsäureundecylester,
3-Nitrobenzylidenacetessigsäuredodecylester,
3-Nitrobenzylidenacetessigsäuretetradecylester,
2-Nitrobenzylidenacetessigsäure-(2-pentoxyethyl)-ester,
3-Nitrobenzylidenacetessigsäure-(ω -chlordecyl)-ester,
3-Nitrobenzylidenacetessigsäure-(ω-hydroxydecyl)-ester,
3-Nitrobenzylidenacetessigsäure-(ω-acetoxydecyl)-ester,
α-Acetyl-β-(pyridyl-3)-acrylsäuredecylester,
α-Acetyl-β-(pyridyl-2)-acrylsäuredecylester,
α-Propionyl-β-(pyridyl-3)-acrylsäuredecylester,
α-Acetyl-β-(benzoxadiazolyl-4)-acrylsäuredecylester,
α-Acetyl-β-(2-methylthio-pyridyl-3)-acrylsäuredecylester.

Die als Ausgangsstoffe verwendeten Enaminocarbonsäureester der Formel (VII) sind literaturbekannt oder können nach literaturbekannten Methoden hergestellt werden
(vgl. A. C. Cope, J. Amer. Chem. Soc. 67, 1017 (1945)).

Als Beispiele seien genannt:
β-Aminocrotonsäurehexylester,
β-Aminocrotonsäureoctylester,
β-Aminocrotonsäuredecylester,
β-Aminocrotonsäuretetradecylester,
β-Aminocrotonsäureisopropylester,
β-Aminocrotonsäurecyclopentylester,
β-Aminocrotonsäurebenzylester,
β-Aminocrotonsäureallylester,
β-Aminocrotonsäure-(2-methoxyethyl)-ester,
β-Aminocrotonsäure-(2-propoxyethyl)-ester,
β-Aminocrotonsäure-(2.2.2-trifluorethyl)-ester,
β-Aminocrotonsäure-(2-chlorethyl)-ester,
β-Aminocrotonsäure-(2-cyanoethyl)-ester,
β-Aminocrotonsäure-(2-hydroxyethyl)-ester,
β-Aminocrotonsäure-(2-acetoxyethyl)-ester,
β-Aminocrotonsäure-(2-phenylethyl)-ester,
β-Aminocrotonsäure-(2-phenoxyethyl)-ester,
β-Aminocrotonsäure-(2-dimethylaminoethyl)-ester,
β-Aminocrotonsäure-(2-N-benzyl-N-methyl-aminoethyl)-ester,
β-Methylaminocrotonsäureethylester,
β-Butylaminocrotonsäureethylester,
β-Benzylaminocrotonsäurethylester.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man

zwischen 20 und 150° C, vorzugsweise zwischen 20 und 100° C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des Yliden-β-ketoesters der Formel (VI) mit einem Mol Enaminocarbonsäureester der Formel (VII) in einem geeigneten Lösungsmittel zur Reaktion gebracht.

**Verfahrensvariante D**

Gemäß Verfahren D wird ein Yliden-β-ketoester der allgemeinen Formel (VI)

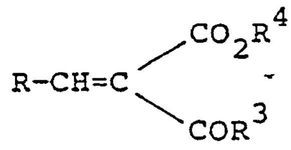

(VI)

mit einem Amin der Formel (IV) und einem β-Ketocarbonsäureester der allgemeinen

$R^5$-$NH_2$,     $R^2$-CO-$CH_2$-$CO_2R^1$
(IV)     (VIII)

Formel (VIII) zur Reaktion gebracht.

Beispiele der als Ausgangsverbindungen verwendeten Yliden-β-ketoester der Formel (VI) sind bereits unter Verfahrensvariante C aufgeführt.

Die erfindungsgemäß verwendbaren Amine der Formel (IV) sind bereits unter Verfahrensvariante B beschrieben.

Die als Ausgangsstoffe verwendeten β-Ketocarbonsäureester der Formel (VIII) sind literaturbekannt oder können nach literaturbekannten Methoden hergestellt werden (z.B. D. Borrmann, "Umsetzung von Diketen mit Alkoholen, Phenolen und Mercaptanen", in Houben-Weyl, Methoden der Organischen Chemie, Vol. VII/4, 230 ff (1968); Y. Oikawa, K. Sugano und O. Yonemitsu, J. Org. Chem. 43, 2087 (1978)).

Als Beispiele seien genannt:
Acetessigsäuremethylester,
Acetessigsäureethylester,
Acetessigsäurehexylester,
Acetessigsäureoctylester,
Acetessigsäuredecylester,
Acetessigsäuretetradecylester,
Acetessigsäureisopropylester,
Acetessigsäureneopentylester,
Acetessigsäurecyclopentylester,
Acetessigsäureallylester,
Acetessigsäure-(2-methoxyethyl)-ester,
Acetessigsäure-(2.2.2-trifluorethyl)-ester,
Acetessigsäure-(2-chlorethyl)-ester,
Acetessigsäure-(2-cyanoethyl)-ester,
Acetessigsäure-(2-acetoxyethyl)-ester,
Acetessigsäurebenzylester,
Acetessigsäure-(2-phenylethyl)-ester,
Acetessigsäure-(2-phenoxyethyl)-ester,
Acetessigsäure-(2-dimethylaminoethyl)-ester,
Acetessigsäure-(2-N-benzyl-N-methyl-aminoethyl)-ester,
Propionylessigsäureethylester,
Propionylessigsäuredecylester.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, insbesondere zwischen 40 und 120° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formeln (VI), (IV) und (VIII) jeweils in molaren Mengen eingesetzt. Das verwendete Amin wird

zweckmäßigerweise im Überschuß von 1 bis 2 Mol zugegeben.

**Verfahrensvarianten E und F**

Gemäß Verfahren E und F wird ein Aldehyd der Formel (IX)

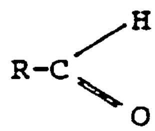

(IX)

entweder (Verfahren E) mit einem Enaminocarbonsäureester der Formel (III) und einem

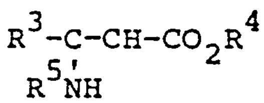

,

$$R^2\text{-CO-CH}_2\text{-CO}_2R^1$$

(III) (VIII)

β-Ketocarbonsäureester der allgemeinen Formel (VIII) oder (Verfahren F) mit einem Enaminocarbonsäureester der allgemeinen Formel (VII)

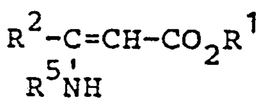

,

$$R^3\text{-CO-CH}_2\text{-CO}_2R^4$$

(VII) (V)

, und einem ß-Ketocarbonsäureester der allgemeinen Formel (V) zur Reaktion gebracht.

Die erfindungsgemäß verwendbaren Enaminocarbonsäureester der allgemeinen Formeln (III) und (VII) sind bereits unter den Verfahrensvarianten A und C angegeben.

Die erfindungsgemäß einsetzbaren β-Ketocarbonsäureester der allgemeinen Formeln (VIII) und (V) wurden bereits unter den Verfahrensvarianten D und B beschrieben.

Die als Ausgangsstoffe verwendeten Aldehyde der Formel (IX) sind literaturbekannt oder können nach literaturbekannten Methoden hergestellt werden (vgl. z.B. E. Mosettig, Org. Reactions VIII, 218 ff (1954)).

Als Beispiele seien genannt:

Benzaldehyd, 2-, 3- oder 4-Phenylbenzaldehyd α- oder β-Naphthylaldehyd, 2-, 3- oder 4-Methylbenzaldehyd, 2- oder 4-n-Butylbenzaldehyd, 2-, 3- oder 4-Isopropyl-benzaldehyd, 2- oder 4-Cyclopropylbenzaldehyd, 2,3-Tetramethylenbenzaldehyd, 3,4-Dioxymethylenbenzaldehyd, 2-, 3- oder 4-Methoxybenzaldehyd, 2-Cyclopropylmethyloxybenzaldehyd, 2-, 3- oder 4-Chlor/Brom/Fluorbenz-aldehyd, 2-, 3- oder 4-Trifluormethylbenzaldehyd, 2-, 3- oder 4-Trifluormethoxybenzaldehyd, 2-, 3- oder 4-Difluormethoxybenzaldehyd, 2-, 3- oder 4-Nitro-benzaldehyd, 2-, 3- oder 4-Cyanobenzaldehyd, 3-Azido-benzaldehyd, 2-, 3- oder 4-Methylthiobenzaldehyd, 2-, 3- oder 4-Methylsulfinylbenzaldehyd, 2-, 3- oder 4-Methylsulfonylbenzaldehyd, 2,3-, 2,4- oder 2,6-Dichlor-benzaldehyd, 2-Fluor-3-chlorbenzaldehyd, 2,4-Dimethyl-benzaldehyd, 2,4- oder 2,6-Dinitrobenzaldehyd, 2-Chlor-6-nitrobenzaldehyd, 4-Chlor-2-nitrobenzaldehyd, 2-Nitro-4-methoxybenzaldehyd, 2-Nitro-4-cyanobenzaldehyd, 2-Chlor-4-cyanobenzaldehyd, 4-Cyano-2-methylbenzaldehyd, 3-Methyl-4-trifluormethylbenzaldehyd, 3-Chlor-2-tri-fluormethylbenzaldehyd, Thiophen-2-aldehyd, Furan-2-aldehyd, Pyrrol-2-aldehyd, Pyrazol-4-aldehyd, Imidazol-2-aldehyd, Oxazol-2-aldehyd, Isoxazol-3-aldehyd, Thiazol-2-aldehyd, Pyridin-2-, 3- oder 4-aldehyd, 6-Methyl-pyridin-2-aldehyd, 2-Methylthio-pyridin-3-aldehyd, Indol-3-aldehyd,

Benzimidazol-2-aldehyd, Benz-oxazol-4-aldehyd, Benzoxadiazol-4-aldehyd, Chinolin-4-aldehyd, Chinazolin-2-aldehyd, Chinoxalin-5-aldehyd.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe jeweils in molaren Mengen eingesetzt.

**Verfahrensvariante G**

Für den Fall, daß X in der allgemeinen Formel I NH bedeutet und $R^2$ oder $R^3$ eine Aminogruppe darstellt, wurde gefunden, daß man die erfindungsgemäßen Verbindungen erhält, wenn man

entweder

Yliden-β-ketoester der allgemeinen Formel (II)

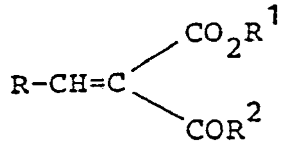 , 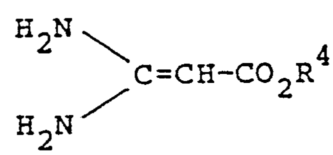

(II) (X)

mit Amidinoessigsäureestern der allgemeinen Formel (X)

oder

Yliden-β-ketoester der allgemeinen Formel (VI)

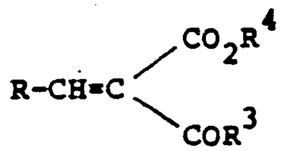 , 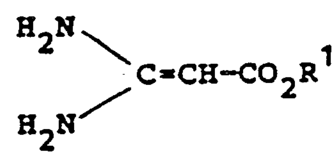

(VI) (XI)

mit Amidinoessigsäureestern der allgemeinen Formel (XI)

zur Reaktion bringt.

Die erfindungsgemäß verwendbaren Yliden-β-ketoester der allgemeinen Formeln (II) und (VI) wurden bereits unter Verfahrensvariante A bzw. C beschrieben.

Die als Ausgangssubstanzen verwendeten Amidinoessigsäureester der Formel (X) sind neu, können aber nach literaturbekannten Methoden hergestellt werden (vgl. z.B. H. Meyer, F. Bossert und H. Horstmann, Liebigs Ann. Chem. 1977, 1895).

Als Beispiele seien genannt:

Amidinoessigsäureheptylester,
Amidinoessigsäureoctylester,
Amidinoessigsäuredecylester,
Amidinoessigsäureundecylester,
Amidinoessigsäuredodecylester,
Amidinoessigsäuretetradecylester,
Amidinoessigsäure-(2-pentoxyethyl)-ester,
Amidinoessigsäure-(2-heptyloxyethyl)-ester,
Amidinoessigsäure-(ω-chlordecyl)-ester,
Amidinoessigsäure-(ω-acetoxydecyl)-ester.

Die als Ausgangssubstanzen verwendeten Amidinoessigsäureester der Formel (XI) sind literaturbekannt oder

können nach bekannten Methoden hergestellt werden (vgl. z.B. H. Meyer, F. Bossert und H. Horstmann, Liebigs Ann. Chem. 1977, 1895).

Als Beispiele seien genannt:
Amidinoessigsäuredecylester,
Amidinoessigsäuretetradecylester,
Amidinoessigsäure-(2-methoxyethyl)-ester,
Amidinoessigsäure-(2-propoxyethyl)-ester,
Amidinoessigsäure-(2-chlorethyl)-ester,
Amidinoessigsäurebenzylester,
Amidinoessigsäure-(2-phenoxyethyl)-ester,
Amidinoessigsäure-(2-dimethylaminoethyl)-ester,
Amidinoessigsäure-(2-N-benzyl-N-methyl-aminoethyl)-ester,
Amidinoessigsäureisopropylester,
Amidinoessigsäurecyclopentylester.

Die Amidine können entweder in freier Form oder in Form ihrer Salze (z.B. Hydrohalogenide) eingesetzt werden. Aus den Salzen werden sie mit basischen Mitteln (z.B. Alkalialkoholaten) freigesetzt.

Als Verdünnungsmittel kommen alle inerten organischen Lösungsmittel in Frage. Hierzu gehören vorzugsweise Alkohole wie Methanol, Ethanol, Propanol; Ether wie Dioxan, Diethylether oder Eisessig, Pyridin, Dimethylformamid, Dimethylsulfoxid oder Acetonitril.

Die Reaktionstemperaturen können in einem größeren Bereich variiert werden. Im allgemeinen arbeitet man zwischen 20 und 250°C, vorzugsweise bei Siedetemperatur des Lösungsmittels.

Die Umsetzung kann bei Normaldruck, aber auch bei erhöhtem Druck durchgeführt werden. Im allgemeinen arbeitet man bei Normaldruck.

Bei der Durchführung des erfindungsgemäßen Verfahrens werden die an der Reaktion beteiligten Stoffe jeweils in molaren Mengen eingesetzt.

**Verfahrensvariante H**

Für den Fall, daß X in der allgemeinen Formel (I) für N-$R^5$ steht, wobei $R^5$ die oben angegebene Bedeutung hat, und $R^2$ oder $R^3$ für die oben angebene Bedeutung hat, es sich als vorteilhaft erwiesen, die entsprechenden erfindungsgemäßen Verbindungen, in denen $R^2$ oder $R^3$ für Acetoxymethyl steht, den Bedingungen einer sauren oder basischen Hydrolyse zu unterwerfen.

Als Säuren kommen in erster Linie Halogenwasserstoffsäuren, Essigsäure oder Ammoniumchlorid in Frage, die Basen haben sich Alkali- und Erdalkalicarbonate als vorteilhaft erwiesen.

Als Verdünnungsmittel kommen Wasser und alle mit Wasser mischbaren inerten organischen Lösungsmittel in Frage. Hierzu gehören vorzugsweise Alkohole wie Methanol oder Ethanol, Ether wie Tetrahydrofuran oder Ethylenglykoldimethylether, Eisessig, Pyridin, Dimethylformamid, Dimethylsulfoxid oder Acetonitril.

Die Reaktionstemperaturen können in einem größeren Bereich variiert werden. Im allgemeinen arbeitet man zwischen 0 und 100°C, vorzugsweise bei Raumtemperatur.

**Verfahrensvariante I**

Für den Fall, daß X in der allgemeinen Formel (I) ein Sauerstoffatom darstellt und $R^2$ oder $R^3$ für eine Aminogruppe steht, wurde gefunden, daß man die erfindungsgemäßen Verbindungen erhält, wenn man entweder

Yliden-β-ketoester der allgemeinen Formel (II)

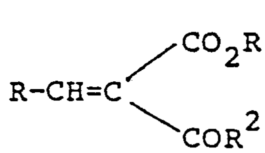
,

$$N{\equiv}C-CH_2-CO_2R^4$$

(II) (XII)

mit Cyanessigsäureestern der allgemeinen Formel (XII)
oder
Yliden-β-ketoester der allgemeinen Formel (VI)

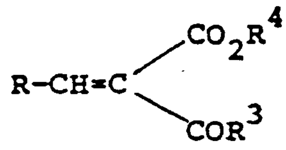

(VI)

$N\equiv C-CH_2-CO_2R^1$

(XIII)

(VI) (XIII)

mit Cyanessigsäureestern der allgemeinen Formel (XIII) zur Reaktion bringt.

Die erfindungsgemäß verwendbaren Yliden-β-ketoester der allgemeinen Formeln (II) und (VI) wurden bereits unter Verfahrensvariante A bzw. C beschrieben.

Die als Ausgangssubstanzen verwendeten Cyanessigsäureester der Formeln (XII) und (XIII) sind bekannt oder konnen nach literaturbekannten Methoden dargestellt werden (siehe Org. Syntheses, Collect. Vol. I, p. 254 (1958)).

Als Beispiele seien genannt:
Cyanessigsäureethylester,
Cyanessigsäurebutylester,
Cyanessigsäureheptylester,
Cyanessigsäuredecylester,
Cyanessigsäureundecylester,
Cyanessigsäuretetradecylester,
Cyanessigsäureisopropylester,
Cyanessigsäurecyclopentylester,
Cyanessigsäurebenzylester,
Cyanessigsäure-(2-methoxyethyl)-ester,
Cyanessigsäure-(2-propoxyethyl)-ester,
Cyanessigsäure-(2-pentyloxyethyl)-ester,
Cyanessigsäure-(2-phenoxyethyl)-ester,
Cyanessigsäure-2.2.2-trifluorethyl)-ester,
Cyanessigsäure-(2-acetoxyethyl)-ester,
Cyanessigsäure-(2-dimethylaminoethyl)-ester,
Cyanessigsäure-(2-N-benzyl-N-methyl-aminoethyl)-ester.

Die Reaktion wird bevorzugt in Gegenwart basischer Katalysatoren, wie z.B. Piperidin, durchgeführt.

Als Verdünnungsmittel kommen alle inerten organischen Losungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe in Gegenwart eines basischen Katalysators jeweils in molaren Mengen eingesetzt.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Je nach Wahl der Ausgangssubstanzen können die er-findungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Raceformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Außer den unten angeführten Herstellungsbeispielen seien folgende erfindungsgemäßen Wirkstoffe genannt:
1,4-Dihydro-2,6-dimethyl-4-(2-nitrophenyl)-pyridin-3,5-dicarbonsäure-heptyl-methyl-ester
1,4-Dihydro-2,6-dimethyl-4-(2-nitrophenyl)-pyridin-3,5-dicarbonsäure-decyl-ethyl-ester
1,4-Dihydro-2,6-dimethyl-4-(2-nitrophenyl)-pyridin-3,5-dicarbonsäure-methyl-undecyl-ester
1,4-Dihydro-2,6-dimethyl-4-(2-nitrophenyl)-pyridin-3,5-dicarbonsäure-heptyl-tetradecyl-ester
1,4-Dihydro-2,6-dimethyl-4-(2-nitrophenyl)-pyridin-3,5-dicarbonsäure-decyl-(2.2.2-trifluorethyl)-ester

2-Amino-1,4-dihydro-6-methyl-4-(2-nitrophenyl)-pyridin-3,5-dicarbonsäure-3-decyl-5-ethyl-ester
1,4-Dihydro-2,6-dimethyl-4-(3-nitrophenyl)-pyridin-3,5-dicarbonsäure-ethyl-heptyl-ester
1,4-Dihydro-2,6-dimethyl-4-(3-nitrophenyl)-pyridin-3,5-dicarbonsäure-decyl-(2.2.2-trifluorethyl)-ester
1,4-Dihydro-2,6-dimethyl-4-(3-nitrophenyl)-pyridin-3,5-dicarbonsäure-(2-N-benzyl-N-methyl-aminoethyl)-decyl-ester
1,4-Dihydro-2-hydroxymethyl-6-methyl-4-(3-nitrophenyl)-pyridin-3,5-dicarbonsäure-2-decyl-5-ethyl-ester
1,4-Dihydro-2,6-dimethyl-4-(pyridyl-3)-pyridin-3 5-dicarbonsäure-decyl-ethyl-ester
1,4-Dihydro-2,6-dimethyl-4-(2.1.3-benzoxadiazolyl-4)-pyridin-3,5-dicarbonsäure-decyl-ethyl-ester
1,4-Dihydro-2,6-dimethyl-4-(2-cyanophenyl)-pyridin-3,5-dicarbonsäure-decyl-ethyl-ester
1,4-Dihydro-2,6-dimethyl-4-(2-trifluormethoxyphenyl)-pyridin-3,5-dicarbonsäure-decyl-ethyl-ester
1,4-Dihydro-2,6-dimethyl-4-(2-methoxyphenyl)-pyridin-3,5-dicarbonsäure-decyl-ethyl-ester
1,4-Dihydro-2,6-dimethyl-4-(2,3-dichlorphenyl)-pyridin-3,5-dicarbonsäure-decyl-ethyl-ester
1,4-Dihydro-2,6-dimethyl-4-(2,3-dichlorphenyl)-pyridin-3,5-dicarbonsäure-decyl-(2.2.2-trifluorethyl)-ester
1,4-Dihydro-2,6-dimethyl-4-(2-trifluormethylphenyl)-pyridin-3,5-dicarbonsäure-methyl-undecylester

Die neuen Verbindungen haben ein breites und vielseitiges pharmakologisches Wirkungsspektrum; mit überraschend langer Wirkungsdauer.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Verbindungen haben stark muskulär-spasmolytische Wirkungen, die an der glatten Muskulatur des Magens, Darmtraktes, des Urogenitaltraktes und des Respirationssystems deutlich werden.

6. Der Vorteil der erfindungsgemäßen Verbindungen liegt in ihrer langanhaltenden, blutdrucksenkenden Wirkung. So wirkt die Verbindung gemäß Beispiel 1 an der Hochdruckratte etwa 24 Stunden blutdrucksenkend. Eine ähnliche Zunahme der Wirkdauer ließ sich bei Hochdruckhunden zeigen. Ein zusätzlicher Vorteil für die therapeutische Anwendung liegt in dem milden Wirkungseintritt der erfindungsgemäßen Verbindungin. Bis zum Auftreten der Maximalwirkung vergehen beim Hund 2-4 und bei der Ratte 4-6 Stunden.

Die erfindungsgemäßen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuss-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Raoline, Tonerden, Talkum, Kreide), synthetische Gesteinsmehle (z.B. hochdisperse Kieselsäure, Silikate), Zucker (z.B. Rohr-,Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinn-gemäß gelten hierbei auch die obigen Ausführungen.

**Herstellungsbeispiele**

**Beispiel 1**

1,4-Dihydro-2,6-dimethyl-4-(5-nitrophenyl)-pyridin-3,5-dicarbonsäure-decyl-ethyl-ester

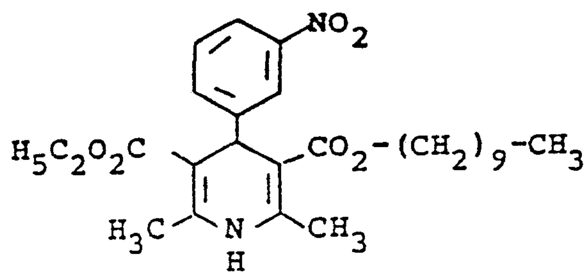

Eine Lösung von 263 g (1 Mol) 2-(3-Nitrobenzyliden)-acetessigsäureethylester und 241 g (1 Mol) 3-Aminocrotonsäuredecylester in 2,4 1 abs. Ethanol wurde 18 Stunden unter Stickstoff zum Sieden erhitzt. Anschließend wurde heiß filtriert und auf ca. die Hälfte des Volumens im Vakuum eigeengt. Die konzentrierte Lösung wurde eisgekühlt, wobei ein dicker Kristallbrei ausfiel. Dieser wurde abgesaugt, aus Ethanol umkristallisiert und nach Waschen mit 0,5 l n-Hexan im Vakuumtrockenschrank bei 60° C getrocknet.

Schmelzpunkt: 106-108° C

Ausbeute: 337 g (69,5 %)

**Beispiel 2**

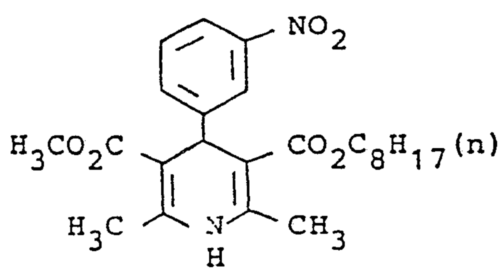

Analog Beispiel 1 wurde durch umsetzung von 2-(3-Nitro-benzyliden)-acetessigsäuremethylester und 3-Aminocrotonsäureoctylester in Ethanol der 1,4-Dihydro-2,6-di methyl-4-(3-mitrophenyl)-pyridin-3,5-dicarbonsäuremethyl-octyl-ester vom Fp. 100° C (Ethanol) erhalten.

Ausbeute: 65% der Theorie

**Beispiel 3**

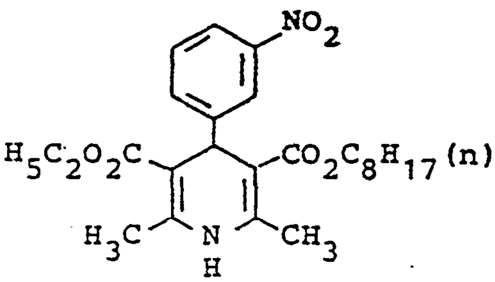

Analog Beispiel 1 wurde durch Umsetzung von 2-(3-Nitro-benzyliden)-acetessigsäureethylester und 3-Aminocrotonsäureoctylester in Ethanol der 1,4-Dihydro-2,6-dimethyl-ester vom Fp. 82°C (Methanol) erhalten.
Ausbeute: 61% der Theorie

**Beispiel4**

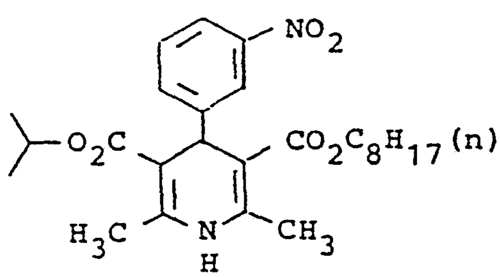

Analog Beispiel 1 wurde durch Umsetzung von 2-(3-Nitro-benzyliden)-acetessigsäureisopropylester mit 5-Aminocrotonsäureoctylester in Ethanol der 1,4-Dihydro-2,6-dimethyl-4-(5-nitrophenyl)-pyridin-5,5-dicarbonsäure-isopropyl-octyl-ester vom Fp. 70°C (Methanol) erhalten.
Ausbeute: 65 % der Theorie

**Beispiel 5**

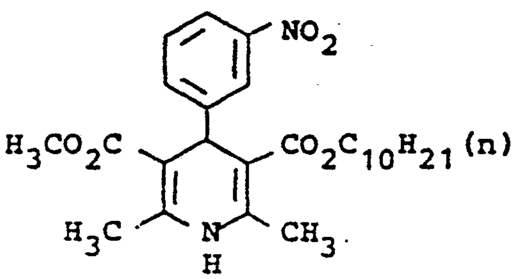

Analog Beispiel 1 wurde durch Umsetzung von 2-(3-Nitro-benzyliden)-acetessigsäuremethylester mit 3-Aminocrotonsäuredecylester in Ethanol der 1,4-Dihydro-2,6-dimethyl-4-(3-nitrophenyl)-pyridin-3,5-dicarbonsäure-decyl-methylester vom Fp. 90°C (Ethanol) erhalten. Ausbeute: 73 % der Theorie

**Beispiel 6**

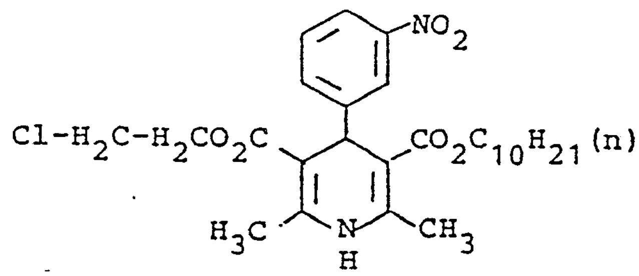

Analog Beispiel 1 wurde durch Umsetzung von 2-(3-Nitro-benzyliden)-acetessigsäure-(2-chlorethyl)-ester mit 3-Aminocrotonsäuredecylester in Ethanol der 1,4-Dihydro-2,6-dimethyl-4-(3-nitrophenyl)-pyridin-3,5-dicarbonsäure-(2-chlorethyl)-decyl-ester vom Fp.: 124° C (Ethanol) erhalten.
Ausbeute: 65 % der Theorie

**Beispiel 7**

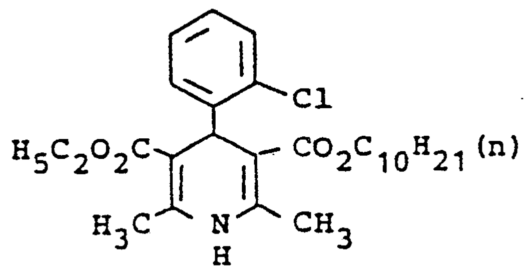

Analog Bespiel 1 wurde durch Umsetzung von 2-(2-Chlor-benzyliden)-acetessigsäureethylester mit 3-Aminocrotonsäuredecylester in Ethanol der 1,4-Dihydro-2,6-dimethyl-4-(2-chlorphenyl)-pyridin-3,5-dicarbonsäure-decyl-ethylester vom Fp: 72° C (Methanol) erhalten.
Ausbeute: 58 % der Theorie.

**Beispiel 8**

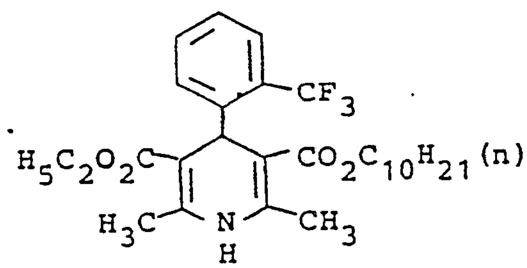

Analog Beispiel 1 wurde durch Umsetzung von 2-(2-Trifluor-methylbenzyliden)-acetessigsäureethylester mit 5-Aminocrotonsäuredecylester in Ethanol der 1,4-Dihydro-2,6-di-methyl-4-(2-trifluormethylphenyl)-pyridin-3,5-dicarbonsäure-decyl-ethyl-ester vom Fp: 76° C (Methanol) erhalten.
Ausbeute: 52 % der Theorie

**Beispiel 9**

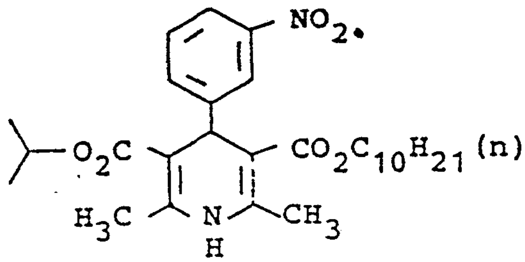

Analog Beispiel 1 wurde durch Umsetzung von 2-(3-Nitro-benzyliden)-acetessigsäureisopropylester mit 3-Aminocrotonsäuredecylester in Ethanol der 1,4-Dihydro-2,6-dimethyl-4-(3-nitrophenyl)-pyridin-3,5-dicarbonsäure-decyl-isopropyl-ester vom Fp: 99° C (Ethanol) erhalten.

Ausbeute: 69 % der Theorie

**Beispiel 10**

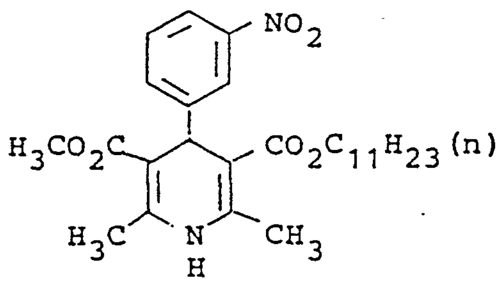

Analog Beispiel 1 wurde durch Umsetzung von 2-(3-Nitro-benzyliden)-acetessigsäuremethylester mit 3-Aminocrotonsäureundecylester in Ethanol der 1,4-Dihydro-2,6-di-methyl-4-(3-nitrophenyl)-pyridin-3,5-dicarbonsäuremethyl-undecyl-ester voa Fp: 85° C (Ethanol) erhalten.

Ausbeute: 78 % der Theorie

**Beispiel 11**

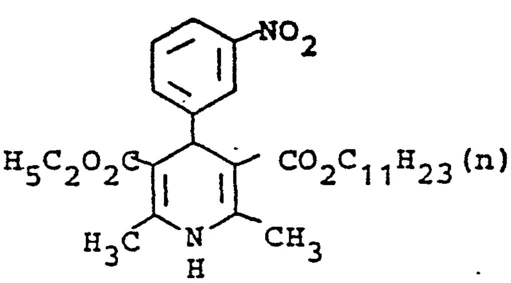

Analog Beispiel 1 wurde durch Umsetzung von 2-(3-Nitro-benzyliden)-acetessigsäureethylester mit 3-Aminocrotonsäureundecylester in Ethanpl der 1,4-Dihydro-2,6-di-methyl-4-(3-nitrophenyl)-pyridin-3,5-dicarbonsäureethyl-undecyl-ester vom Fp: 107° C (Ethanol) erhalten.

Ausbeute: 75 % der Theorie

**Beispiel 12**

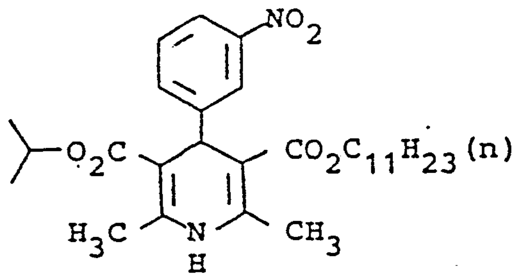

Analog Beispiel 1 wurde durch Umsetzung von 2-(3-Nitro-benzyliden)-acetessigsäureisopropylester mit 3-Aminocrotonsäureundecylester in Ethanol der 1,4-Dihydro-2,6-dimethyl-4-(3-nitrophenyl)-pyridin-3,5-dicarbonsäureisopropyl-undecyl-ester vom Fp: 108° C (Ethanol) erhalten.
Ausbeute: 69 % der Theorie

**Beispiel 13**

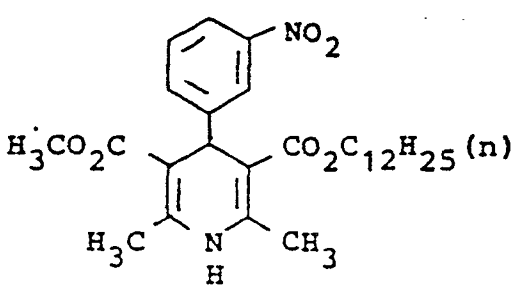

Analog Beispiel 1 wurde durch Umsetzung von 2-(3-Nitro-benzyliden)-acetessigsäuremethylester mit 3-Aminocrotonsäuredodecylester in Ethanol der 1,4-Dihydro-2,6-di-methyl-4-(3-nitrophenyl)-pyridin-3,5-dicarbonsäuredodecyl-methyl-ester vom Fp: 94° C (Ethanol) erhalten.
Ausbeute: 66 % der Theorie

**Beispiel 14**

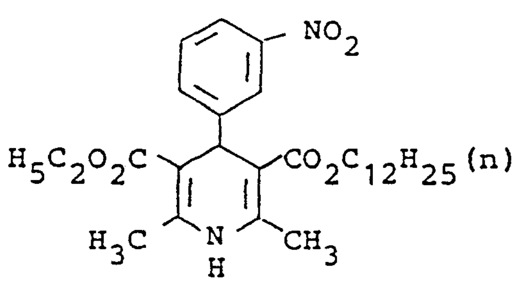

Analog Beispiel 1 wurde durch Umsetzung von 2-(3-Nitro-benzyliden)-acetessigsäureethylester mit 3-Aminocrotonsauredodecylester in Ethanol der 1,4-Dihydro-2,6-di-methyl-4-(3-nitrophenyl)-pyridin-3,5-dicarbonsäuredodecyl-ethyl-ester vom Fp.: 100° C (Ethanol) erhalten.
Ausbeute: 76 % der Theorie

**Beispiel 15**

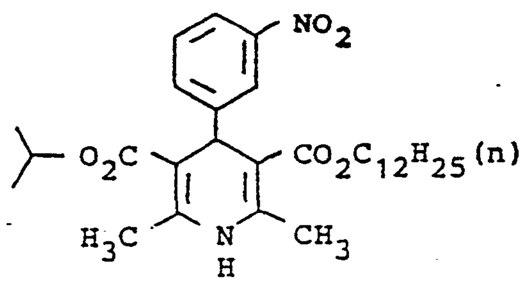

Analog Beispiel 1 wurde durch Umsetzung von 2-(3-Nitro-benzyliden)-acetessigsäureisopropylester mit 3-Aminocrotonsäuredodecylester in Ethanol der 1,4-Dihydro-2,6-dimethyl-4-(3-nitrophenyl)-pyridin-3,5-dicarbonsäuredodecyl-isopropyl-ester vom Fp.: 100° C (Ethanol) erhalten.
Ausbeute: 60 % der Theorie

**Beispiel 16**

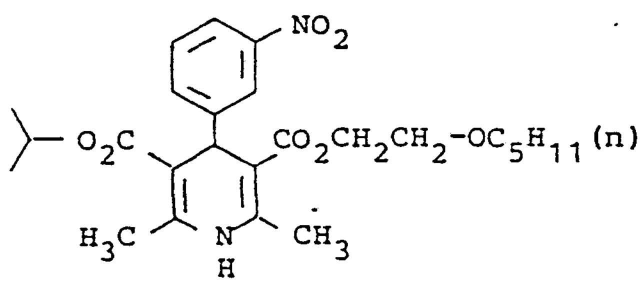

Analog Beispiel 1 wurde durch Umsetzung von 2-(3-Nitro-benzyliden)-acetessigsäureisopropylester mit 3-Amino-crotonsäure-(2-pentyloxyethyl)-ester in Isopropanol der 1,4-Dihydro-2,6-dimethyl-4-(3-nitrophenyl)-pyridin-3,5-dicarbonsäure-isopropyl-(2-pentyloxyethyl)-ester vom Fp.: 75° C (Ether/Petrolether) erhalten.
Ausbeute: 65 % der Theorie

**Beispiel 17**

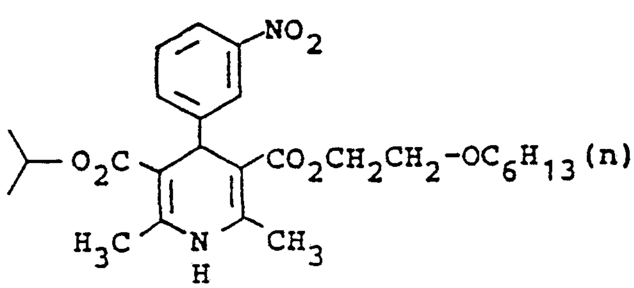

Analog Beispiel 1 wurde durch Umsetzung von 2-(3-Nitro-benzyliden)-acetessigsäureisopropylester mit 3-Amino-crotonsäure-(2-hexyloxyethyl)-ester in Isopropanol der 1,4-Dihydro-2,6-dimethyl-4-(3-nitrophenyl)-pyridin-3,5-dicarbonsäure-(2-hexyloxyethyl)-isopropylester vom Fp.: 83° C (Ether/Petrolether) erhalten.
Ausbeute: 80 % der Theorie

**Beispiel 18**

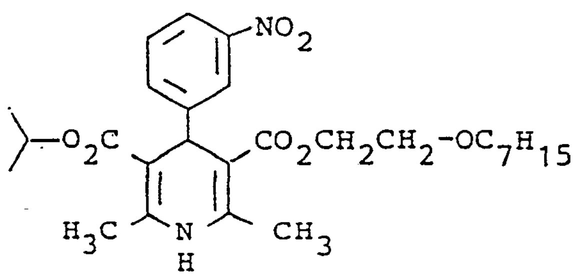

Analog Beispiel 1 wurde durch Umsetzung von 2-(3-Nitro-benzyliden)-acetessigsäureisopropylester mit 3-Amino-crotonsäure-(2-heptyloxyethyl)-ester in Isopropanol der 1,4-Dihydro-2,6-dimethyl-4-(3-nitrophenyl)-pyridin-3,5-dicarbonsäure-(2-heptyloxyethyl)-isopropylester vom Fp: 80°C (Ether/Petrolether) erhalten.
Ausbeute: 72 % der Theorie

**Beispiel 19**

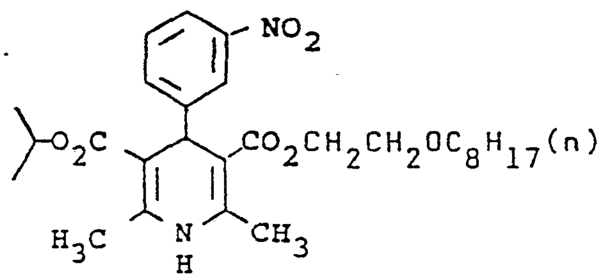

Analog Beispiel 1 wurde durch Umsetzung von 2-(3-Nitro-benzyliden)-acetessigsäureisopropylester mit 3-Amino-crotonsäure-(2-octyloxyethyl)-ester in Isopropanol der 1,4-Dihydro-2,6-dimethyl-4-(3-nitrophenyl)-pyridin-3,5-dicarbonsäure-isopropyl-(2-octyloxyethyl)-ester vom Fp.: 89°C (Ether/Petrolether) erhalten.
Ausbeute: 76 % der Theorie

**Beispiel 20**

2-Amino-1,4-dihydro-6-methyl-4-(3-nitrophenyl)-pyridin-3,5-dicarbonsäure-5-decyl-3-ethyl-ester

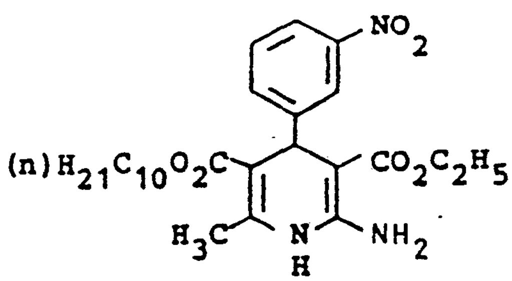

In einer Lösung von 18,8 g (50mMol) 2-(3-Nitrobenzyliden)-acetessigsäuredecylester und 8,3 g (50 mMol) Amidinoessigsäureethylester-hydrochlorid in 250 ml Ethanol wurden unter Rühren und bei Raumtemperatur eine Lösung von 1,15 g (50 mMol) Natrium in 100 ml Ethanol zugetropft. An-schließend wurde 15 Minuten zum Sieden erhitzt. Nach Stehen über Nacht wurde vom ausgefallenen Natriumchlorid abfiltriert, das Filtrat im Vakuum eingeengt und der ölige Rückstand durch Verreiben mit Ether/Petrolether zur Kristallisation gebracht. Das Rohprodukt wurde abgesaugt und aus Ethanol umkristallisiert, Fp.: 190-191°C.
Ausbeute: 65 % der Theorie

**Beispiel 21**

2-Amino-6-methyl-4-(3-nitrophenyl)-4H-pyran-3,5-dicar-bonsäure-5-decyl-3-ethyl-ester

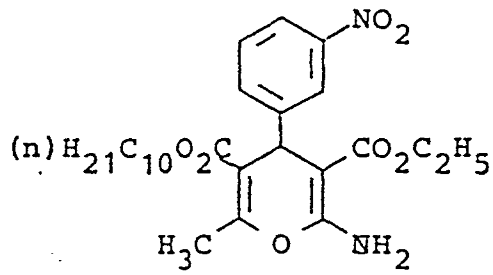

Eine Lösung von 5,7 g (15 mMol) 2-(3-Nitrobenzyliden)-acetessigsäuredecylester und 1,8 g (15 mMol) Cyanessigsaureethylester in 15 ml Ethanol wurde in Gegenwart von 0,3 ml Piperidin 4 Stunden zum Sieden erhitzt. Anschlie-ßend wurde das Lösungsmittel abdestilliert und der ölige Rückstand nach säulenchromatographischer Reinigung ($SiO_2$, Chloroform) zur Kristallisation gebracht, Fp.: 100°C, Ausbeute: 3,3 g (45 %)

**Patentansprüche**

1. Verbindungen der allgemeinen Formel (I)

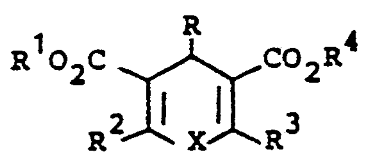

(I)

in welcher

R für Phenyl, Naphthyl oder für Thienyl Furyl Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxymethylen, Halogen, Trifluormethyl, Trifluormethoxy, Difluormethoxy, Tetrafluorethoxy, Nitro, Cyano, Azido oder $SO_m$-Alkyl, worin m eine Zahl von 0 bis 2 bedeutet und Alkyl vorzugsweise 1 bis 4 Kohlenstoffatome enthält, substituiert sein können,

$R^1$ für einen geradkettigen, verzweigten oder cyclischen, gesättigten oder ungesättigten Kohlenwasserstoffrest mit bis zu 20 Kohlenstoffatomen steht, der gegebenenfalls durch 1 Sauerstoffatom in der Kette unterbrochen ist und/oder der gegebenenfalls substituiert ist durch Halogen, Cyano, Hydroxy, Acetoxy, Phenyl, Phenoxy oder eine Aminogruppe, die ihrerseits durch 2 gleiche oder verschiedene Substituenten aus der Gruppe Alkyl mit 1 bis 4 Kohlenstoffatomen, Phenyl oder Benzyl substituiert ist,

$R^4$ für einen geradkettigen, verzweigten oder cyclischen, gesättigten oder ungesättigten aliphatischen Kohlenwasserstoffrest mit 7 bis 14 Kohlenstoffatomen steht, der gegebenenfalls durch ein Sauerstoffatom in der Kette unterbrochen ist und/ oder der gegebenenfalls durch Halogen, Hydroxy oder Acetoxy substituiert ist,

$R^2$ und $R^3$ gleich oder verschieden sind und jeweils für Wasserstoff, einen geradkettigen oder verzweigten Alkylrest mit bis zu 4 Kohlenstoffatomen, einen Thenylrest oder einen Benzylrest stehen, oder einer der Substituenten $R^2$ oder $R^3$ fur eine Hydroxymethyl-, Acetoxymethyl- oder Aminogruppe steht, wobei der andere Substituent die oben angegebene Bedeutung besitzt, und

X für N-$R^5$ steht, wobei $R^5$ für Wasserstoff, einen geradkettigen oder verzweigten, gegebenenfalls durch ein Sauerstoffatom unterbrochenen Alkylrest mit bis zu 4 Kohlenstoffatomen, einen Morpholinoethylrest, einen Phenylrest oder einen Benzylrest, steht, oder

für ein Sauerstoffatom steht, wobei dann einer der Substituenten $R^2$ oder $R^3$ immer eine Aminogruppe bedeutet.

2. Verbindungen der allgemeinen Formel (I) gemäß Anspruch 1, in welcher

R für Phenyl, Pyridyl, Thienyl oder Furyl, steht wobei der Phenylring gegebenenfalls durch ein oder zwei gleiche oder verschiedene Substituenten aus der Gruppe Phenyl, Alkyl mit 1 bis 4 Kohlenstoffatomen, Alkoxy mit 1 bis 2 Kohlenstoffatomen, Fluor, Chlor, Brom, Trifluormethyl, Trifluormethoxy, Difluormethoxy, Nitro, Cyano,

Azido oder Alkylmercapto mit 1 bis 4 Kohlenstoffatomen im Alkylrest substituiert ist,

$R^1$ für einen geradkettigen verzweigten oder cyclischen, gesättigten oder ungesättigten Kohlenwasserstoffrest mit bis zu 14 Kohlenstoffatomen steht der gegebenenfalls durch ein Sauerstoffatom unterbrochen ist und/oder der gegebenenfalls substituiert ist durch Fluor, Chlor, Cyano, Hydroxy oder Amino,

$R^4$ für einen geradkettigen, verzweigten oder cyclischen, gesättigten oder ungesättigten aliphatischen Kohlenwasserstoffrest mit 7 bis 12 Kohlenstoffatomen steht, der gegebenenfalls durch ein Sauerstoffatom in der Kette unterbrochen ist und/oder der gegebenenfalls durch Fluor, Chlor, Hydroxy oder Acetoxy substituiert ist,

$R^2$ und $R^3$ gleich oder verschieden sind und jeweils für Wasserstoff, Alkyl mit 1 bis 2 Kohlenstoffatomen, Phenyl oder Benzyl stehen,

oder

einer der Substituenten $R^2$ oder $R^3$ für eine Hydroxymethyl-, Acetoxymethyl- oder Aminogruppe steht, wobei der andere Substituent die vorher angegebene Bedeutung besitzt,

X fur N-$R^5$ steht, wobei $R^5$ für Wasserstoff, einen Alkylrest mit bis zu 4 Kohlenstoffatomen der gegegebenenfalls durch ein Sauerstoffatom unterbrochen ist, einen Morpholinoalkylrest mit 1 bis 4 Kohlenstoffatomen im Alkylteil, einen Phenylrest oder einen Benzylrest steht,

oder

für ein Sauerstoffatom steht, wobei dann einer der Substituenten $R^2$ oder $R^3$ immer eine Aminogruppe bedeutet.

3. Verbindungen der allgemeinen Formel (I) gemäß Anspruch 1, in welcher

R für einen Phenylrest steht, der ein oder zwei gleiche oder verschiedene Substituenten aus der Gruppe Chlor, Trifluormethyl, Nitro oder Cyano enthält,

oder für einen Benzoxadiazolylrest steht,

$R^1$ einen geradkettigen oder verzweigten Kohlenwasserstoffrest mit bis zu 7 Kohlenstoffatomen darstellt, der gegebenenfalls durch ein Sauerstoffatom in der Kette unterbrochen ist und/oder der gegebenenfalls durch Fluor oder Chlor oder eine Aminogruppe substituiert ist, die ihrerseits durch zwei Methylgruppen oder durch eine Methyl- und eine Benzylgruppe substituiert sein kann,

$R^4$ einen geradkettigen, verzweigten oder cyclischen, gesättigten oder ungesättigten aliphatischen Kohlenwasserstoffrest mit sieben bis zwölf Kohlenstoffatomen bedeutet, der gegebenenfalls durch ein Sauerstoffatom in der Kette unterbrochen ist oder der gegebenenfalls durch Fluor, Chlor oder Hydroxy substituiert ist,

$R^2$ und $R^3$ jeweils für Wasserstoff oder einen Alkylrest mit 1 bis 2 Kohlenstoffatomen stehen, oder eine der Substituente $R^2$ oder $R^3$ für die Aminogruppe steht, wobei der andere Substituent die oben angegebene Bedeutung hat, und

X für N-$R^5$ steht, wobei $R^5$ ein Wasserstoffatom oder einen Alkylrest mit bis zu 3 Kohlenstoffatomen darstellt,

oder

für ein Sauerstoffatom steht, wobei ein der Substituenten $R^2$ oder $R^3$ immer eine Aminogruppe darstellt.

4. Verbindungen der allgemeinen Formel (I) gemäβ Anspruch 1, in welcher

$R^4$ einen geradkettigen, verzweigten oder cyclischen gesättigten oder ungesättigten aliphatischen Kohlenwasserstoffrest mit 10 Kohlenstoffatomen bedeutet, der gegebenenfalls durch ein Sauerstoffatom in der Kette unterbrochen ist oder der gegebenenfalls durch Fluor, Chlor. oder Hydroxy substituiert ist, und

die übrigen Substituenten, die in Anspruch 2 angegebene Bedeutung besitzen.

5. Verbindungen der allgemeinen Formel (I) gemäß Anspruch 1 zur Verwendung bei der Bekämpfung von Kreislauferkrankungen.

6. Verfahren zur Herstellung von Verbindungen der allgemeinen Formel (I)

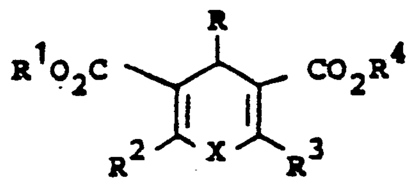

(I)

in welcher

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxymethylen, Halogen, Trifluormethyl, Trifluormethoxy, Difluormethoxy, Tetrafluorethoxy, Nitro, Cyano, Azido oder $SO_m$-Alkyl, worin m eine Zahl von 0 bis 2 bedeutet und Alkyl vorzugsweise 1 bis 4 Kohlenstoffatome enthält, substituiert sein können,

$R^1$ für einen geradkettigen, verzweigten oder cyclischen, gesättigten oder ungesättigten Kohlenwasserstoffrest mit bis zu 20 Kohlenstoffatomen steht, der gegebenenfalls durch 1 Sauerstoffatom in der Kette unterbrochen ist

und/oder der gegebenenfalls substituiert ist durch Halogen, Cyano, Hydroxy, Acetoxy, Phenyl, Phenoxy oder eine Aminogruppe, die ihrerseits durch 2 gleiche oder verschiedene Substituenten aus der Gruppe Alkyl mit 1 bis 4 Kohlenstoffatomen, Phenyl oder Benzyl substituiert ist,

$R^4$ für einen geradkettigen, verzweigten oder cyclischen, gesättigten oder ungesättigten aliphatischen Kohlenwasserstoffrest mit 7 bis 14 Kohlenstoffatomen steht, der gegebenenfalls durch ein Sauerstoffatom in der Kette unterbrochen ist und/ oder der gegebenenfalls durch Halogen, Hydroxy oder Acetoxy substituiert ist,

$R^2$ und $R^3$ gleich oder verschieden sind und jeweils für Wasserstoff, einen geradkettigen oder verzweigten Alkylrest mit bis zu 4 Kohlenstoffatomen, einen Phenylrest oder einen Benzylrest stehen, oder einer der Substituenten $R^2$ oder $R^3$ für eine Hydroxymethyl-, Acetoxymethyl- oder Aminogruppe steht, wobei der andere Substituent die oben angegebene Bedeutung besitzt, und

X für N-$R^5$ steht, wobei $R^5$ für Wasserstoff, einen geradkettigen oder verzweigten, gegebenenfalls durch ein Sauerstoffatom unterbrochenen Alkylrest mit bis zu 4 Kohlenstoffatomen, einen Morpholinoethylrest, einen Phenylrest oder einen Benzylrest, steht,

oder

für ein Sauerstoffatom steht, wobei dann einer der Substituenten $R^2$ oder $R^3$ immer eine Aminogruppe bedeutet,

indem man: A)

Yliden-β-ketoester der allgemeinen Formel (II)

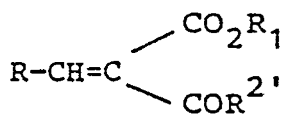

(II)

in welcher

R, $R^1$ und $R^{2'}$ die oben angegebene Bedeutung haben,

mit Enaminocarbonsäureestern der allgemeinen Formel (III)

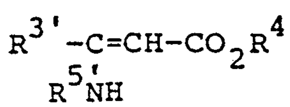

(III)

in welcher

$R^{3'}$, $R^5$ und $R^4$ die oben angegebene Bedeutung haben,

gegebenenfalls in Gegenwart inerter organischer Lösungsmittel umsetzt,

oder

B)

Yliden-β-ketoester der allgemeinen Formel (II)

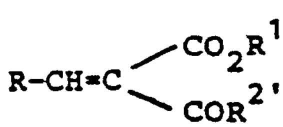

(II)

in welcher

R,$R^1$ und $R^{2'}$ die oben angegebene Bedeutung haben,

mit Aminen der allgemeinen Formel (IV) und β-Ketocarbonsäureestern der allgemeinen Formel (V)

$R^5$-$NH_2$ $R^{3'}$-CO-$CH_2$-$CO_2R^4$

(IV) (V)

in welchen

$R^5$, $R^{3'}$ und $R^4$ die oben angegebene Bedeutung haben, gegebenenfalls in Gegenwart inerter organischer Lösungsmittel umsetzt,

oder

C)

Yliden-β-ketoester der allgemeinen Formel (VI)

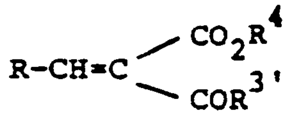

(VI)

in welcher R, $R^{3'}$ und $R^4$ die oben angegebene Bedeutung haben, mit Enaminocarbonsäureestern der allgemeinen Formel (VII)

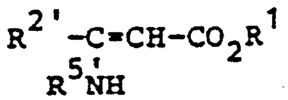

(VII)

in welcher $R^{2'}$, $R^5$ und $R^1$ die oben angegebene Bedeutung haben, gegebenenfalls in Gegenwart inerter organischer Lösungsmittel zur Reaktion bringt, oder

D)

Yliden-β-ketoester der allgemeinen Formel (VI)

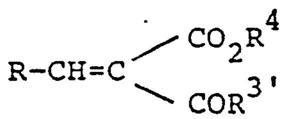

(VI)

in welcher R, $R^{3'}$ und $R^4$ die oben angegebene Bedeutung haben, mit Aminen der allgemeinen Formel (IV)

$R^5$ -$NH_2$ (IV)

in welcher $R^5$ die oben angegebene Bedeutung hat, und β-Ketocarbonsäureestern der allgemeinen Formel (VIII)

$R^{2'}$-CO-$CH_2$-$CO_2R^1$ (VIII)

in welcher $R^{2'}$ und $R^1$ die oben angegebene Bedeutung haben, gegebenenfalls in Gegenwart inerter organischer Lösungsmittel umsetzt oder

E)

Aldehyde der allgemeinen Formel (IX)

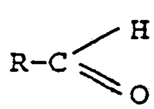

(IX)

in welcher R die oben angegebene Bedeutung hat, mit Enaminocarbonsäureestern der allgemeinen Formel (III)

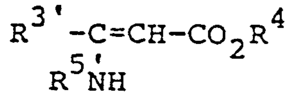

(III)

in welcher
$R^{3'}$, $R^4$ und $R^5$ die oben angegebene Bedeutung haben,
und mit β-Ketocarbonsäureestern der allgemeinen Formel (VIII)
$R^{2'}-CO-CH_2-CO_2R^1$ (VIII)
in welcher
$R^1$ und $R^{2'}$ die oben angegebene Bedeutung haben,
gegebenenfalls in Gegenwart inerter organischer Lösungsmittel umsetzt,
oder
F)
Aldehyde der allgemeinen Formel (IX)

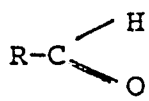

(IX)

in welcher
R die oben angegebene Bedeutung hat,
mit Enaminocarbonsäureestern der allgemeinen Formel (VII)

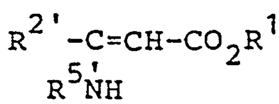

(VII)

in welcher $R^1$, $R^{2'}$ und $R^5$ die oben angegebene Bedeutung haben
und mit β-Ketocarbonsäureestern der allgemeinen Formel (V)
$R^{3'}-CO-CH_2-CO_2R^4$ (V)
in welcher $R^{3'}$ und $R^4$ die oben angegebene Bedeutung haben,
gegebenenfalls in Gegenwart inerter organischer Lösungsmittel zur Reaktion bringt,
oder
G)
für den Fall, daß X in der Formel (I) NH bedeutet, und $R^2$ oder $R^3$ eine Aminogruppe darstellen, indem man
1. Yliden-β-ketoester der allgemeinen Formel (II)

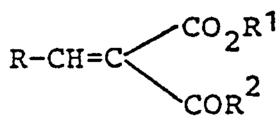

(II)

in welcher
R, $R^1$ und $R^2$ die oben angegebene Bedeutung haben, aber $R^2$ nicht für eine Amino- oder Hydroxymethylgruppe steht,
mit Amidinoessigsäureestern der allgemeinen Formel (X)

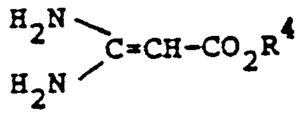

(X)

in welcher $R^4$ die oben angegebene Bedeutung hat, gegebenenfalls in Gegenwart inerter organischer Lösungsmittel umsetzt oder

2. Yliden-β-ketoester der allgemeinen Formel (VI)

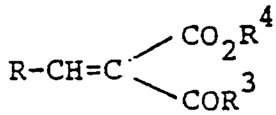

(VI)

in welcher $R$, $R^3$ und $R^4$ die oben angegebene Bedeutung haben, aber $R^3$ nicht für eine Amino- oder Hydroxymethylgruppe steht, mit Amidinoessigsäureestern der allgemeinen Formel (XI)

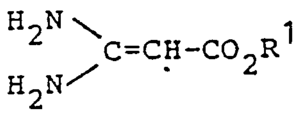

(XI)

in welcher $R^1$ die oben angegebene Bedeutung hat, gegebenenfalls in Gegenwart inerter organischer Lösungsmittel zur Reaktion bringt, oder

H)

für den Fall, daß X in der allgemeinen Formel (I) für N-$R^5$ steht, wobei $R^5$ die oben angegebene Bedeutung hat, und R oder $R^3$ für die Hydroxymethylgruppe stehen, indem man die entsprechenden erfindungsgemäßen Verbindungen, bei denen $R^2$ dder $R^3$ Acetoxymethyl bedeuten, den Bedingungen einer sauren oder basischen Hydrolyse unterwirft, wobei die Acetoxymethylgruppe in eine Hydroxymethylgruppe überführt wird, oder

I)

für den Fall, daß X in der allgemeinen Formel (I) ein Sauerstoffatom darstellt und $R^2$ oder $R^3$ für eine Aminogruppe steht, indem man

1. Yliden-β-ketoester der allgemeinen Formel (II)

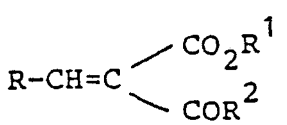

(II)

in welcher $R$, $R^1$ und $R^2$ die oben angegebene Bedeutung haben, aber $R^2$ nicht für die Amino- oder Hydroxymethylgruppe steht, mit Cyanessigsäureestern der allgemeinen Formel (XII)

$N{\equiv}C\text{-}CH_2\text{-}CO_2R^4$ (XII)

in welcher $R^4$ die oben angegebene Bedeutung hat, gegebenenfalls in Gegenwart organischer Lösungsmittel umsetzt, oder

2. Yliden-β-ketoester der allgemeinen Formel (VI)

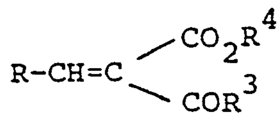

(VI)

in welcher
R, $R^4$ und $R^3$ die oben angegebene Bedeutung haben, aber $R^3$ nicht für die Amino- oder Hydroxymethylgruppe steht,
mit Cyanessigsäureestern der allgemeinen Formel (XIII)
$N\equiv C\text{-}CH_2\text{-}CO_2R^1$ (XIII)
in welcher
$R^1$ die oben angegebene Bedeutung hat,
gegebenenfalls in Gegenwart organischer Lösungsmittel zur Reaktion bringt,
und die erhaltenen Verbindungen der allgemeinen Formel (I) gegebenenfalls in ihre Additionsalze überführt.

7. Arzneimittel enthalten mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

9. Arzneimittel mit langanhaltender Kreislaufwirkung enthaltend mindestens eine Verbindung der allgemeinen Formel (I) gemäß Anspruch 1.

10. Verwendung von Verbindungen der allgemeinen Formel (I), gemäß Anspruch 1 zur Herstellung von Blutdruck senkenden Mitteln.

**Claims**

1. Compounds of the general formula (I)

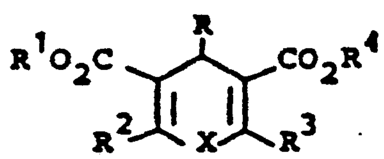

(I)

in which
R represents phenyl, naphthyl or thienyl, furyl, pyrryl, pyrazolyl imidazolyl, oxazolyl, isoxazolyl, thiazolyl, pyridyl, pyridazinyl, pyrimidyl, pyrazinyl, indolvl, benzimidazolyl, benzoxazolyl, benzisoxazolyl,benzoxadiazolyl quinolyl, isoquinolyl, quinazolyl or quinoxalyl, it being possible for the ring systems mentioned each to be substituted by 1 or 2 identical or different substituents from the group comprising phenyl, straight-chain or branched alkyl with 1 to 8 carbon atoms, cycloalkyl with 3 to 7 carbon atoms, alkoxy with 1 to 4 carbon atoms, tri-, tetra- and pentamethylene, dioxymethylene, halogen, trifluoromethyl, trifluoromethoxy, difluoromethoxy, tetrafluoroethoxy, nitro, cyano, azido or $SO_m$ -alkyl, wherein m denotes a number from 0 to 2 and alkyl preferably contains 1 to 4 carbon atoms,
$R^1$ represents a straight-chain, branched or cyclic, saturated or unsaturated hydrocarbon radical with up to 20 carbon atoms, which is optionally interrupted by 1 oxygen atom in the chain and/or which is optionally substituted by halogen, cyano, hydroxyl, acetoxy, phenyl, phenoxy or an amino group which in turn is substituted by 2 identical or different substituents from the group comprising alkyl with 1 to 4 carbon atoms, phenyl or benzyl,
$R^4$ represents a straight-chain, branched or cyclic, saturated or unsaturated aliphatic hydrocarbon radical with 7 to 14 carbon atoms which is optionally interrupted by an oxygen atom in the chain and/or which is optionally substituted by halogen, hydroxyl or acetoxy,
$R^2$ and $R^3$ are identical or different and each represent hydrogen, a straight-chain or branched alkyl radical with up to 4 carbon atoms, a phenyl radical or a benzyl radical, or one of the substituents $R^2$ or $R^3$ represents a hydroxymethyl, acetoxymethyl or amino group, the other substituent having the abovementioned meaning,
and
X represents N-$R^5$, wherein $R^5$ represents hydrogen, a straight-chain or branched alkyl radical which has up to 4 carbon atoms and is optionally interrupted by an oxygen atom, a morpholinoethyl radical, a phenyl radical or a benzyl radical, or represents an oxygen atom, in which case one of the substituents $R^2$ or $R^3$ always denotes an

amino group.

2. Compounds of the general formula (I) according to Claim 1, in which

R represents phenyl, pyridyl, thienyl or furyl, the phenyl ring optionally being substituted by one or two identical or different substituents from the group comprising phenyl, alkyl with 1 to 4 carbon atoms, alkoxy with 1 to 2 carbon atoms, fluorine, chlorine, bromine, trifluoromethyl, trifluoromethoxy, difluoromethoxy, nitro, cyano, azido or alkylmercapto with 1 to 4 carbon atoms in the alkyl radical,

$R^1$ represents a straight-chain, branched or cyclic, saturated or unsaturated hydrocarbon radical with up to 14 carbon atoms which is optionally interrupted by an oxygen atom and/or which is optionally substituted by fluorine, chlorine, cyano, hydroxyl or amino,

$R^4$ represents a straight-chain, branched or cyclic, saturated or unsaturated aliphatic hydrocarbon radical with 7 to 12 carbon atoms which is optionally interrupted by an oxygen atom in the chain and/or which is optionally substituted by fluorine, chlorine, hydroxyl or acetoxy,

$R^2$ and $R^3$ are identical or different and each represent hydrogen, alkyl with 1 to 2 carbon atoms, phenyl or benzyl,

or

one of the substituents $R^2$ or $R^3$ represents a hydroxymethyl, acetoxymethyl or amino group, the other substituent having the previously mentioned meaning,

X represents N-$R^5$, wherein $R^5$ represents hydrogen, an alkyl radical with up to 4 carbon atoms which is optionally interrupted by an oxygen atom, a morpholinoaökyl radical interrupted by an oxygen atom, a morpholinoalkyl radical having 1 to 4 carbon atoms in the alkyl part, a phenyl radical or a benzyl radical,

or

represents an oxygen atom, in which case one of the substituents $R^2$ or $R^3$ always denotes an amino group.

3. Compounds of the general formula (I) according to Claim 1, in which

R represents a phenyl radical which contains one or two identical or different substituents from the group comprising chlorine, trifluoromethyl, nitro or cyano,

or represents a benzoxadiazolyl radical,

$R^1$ represents a straight-chain or branched hydrocarbon radical with up to 7 carbon atoms which is optionally interrupted by an oxygen atom in the chain and/or which is optionally substituted by fluorine or chlorine or an amino group, which in turn can be substituted by two methyl groups or by a methyl and a benzyl group,

$R^4$ denotes a straight-chain, branched or cyclic, saturated or unsaturated aliphatic hydrocarbon radical with seven to twelve carbon atoms, which is optionally interrupted by an oxygen atom in the chain or which is optionally substituted by fluorine, chlorine or hydroxyl,

$R^2$ and $R^3$ each represent hydrogen or an alkyl radical with 1 to 2 carbon atoms,

or one of the substituents $R^2$ or $R^3$ represents the amino group, the other substituent having the abovementioned meaning, and

X represents N-$R^5$, wherein $R^5$ represents a hydrogen atom or an alkyl radical with up to 3 carbon atoms,

or

represents an oxygen atom, one of the substituents $R^2$ or $R^3$ always representing an amino group.

4. Compounds of the general formula (I) according to Claim 1, in which

$R^4$ denotes a straight-chain, branched or cyclic saturated or unsaturated aliphatic hydrocarbon radical with 10 carbon atoms, which is optionally interrupted by an oxygen atom in the chain or which is optionally substituted by fluorine, chlorine or hydroxyl, and

the remaining substituents have the meaning given in Claim 2.

5. Compounds of the general formula (I) according to Claim 1 for use in combating cardiovascular diseases.

6. Process for the preparation of compounds of the general formula (I)

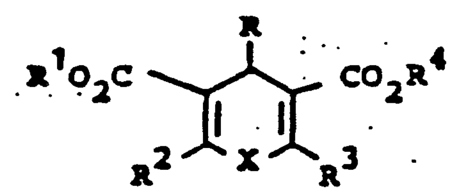

(I)

in which

R represents Phenyl, naphthyl or thienyl, furyl, pyrryl, pyrazoly] imidazolyl, oxazolyl, isoxazolyl, thiazolyl, Pyridyl, pyridazinyl, pyrimidyl, quinolyl, isoquinolyl, quinazolyl or quinoxalyl, it being possible for the ring systems mentioned each to be substituted by 1 or 2 identical or different substituents from the group comprising phenyl, straight-chain or branched alkyl with 1 to 8 carbon atoms, cycloalkyl with 3 to 7 carbon atoms, alkoxy with 1 to 4 carbon atoms, tri-, tetra- and pentamethylene, dioxymethylene, halogen, trifluoromethyl, trifluoromethoxy, difluoromethoxy, tetrafluoroethoxy, nitro, cyano, azido or $SO_m$-alkyl, wherein m denotes a number from 0 to 2 and alkyl preferably contains 1 to 4 carbon atoms,

$R^1$ represents a straight-chain, branched or cyclic, saturated or unsaturated hydrocarbon radical with up to 20 carbon atoms, which is optionally interrupted by 1 oxygen atom in the chain and/or which is optionally

substituted by halogen, cyano, hydroxyl, acetoxy, phenyl, phenoxy or an amino group which in turn is substituted by 2 identical or different substituents from the group comprising alkyl with 1 to 4 carbon atoms, phenyl or benzyl,

$R^4$ represents a straight-chain, branched or cyclic, saturated or unsaturated aliphatic hydrocarbon radical with 7 to 14 carbon atoms which is optionally interrupted by an oxygen atom in the chain and/or which is optionally substituted by halogen, hydroxyl or acetoxy,

$R^2$ and $R^3$ are identical or different and each represent hydrogen, a straight-chain or branched alkyl radical with up to 4 carbon atoms, a phenyl radical or a benzyl radical, or one of the substituents $R^2$ or $R^3$ represents a hydroxymethyl, acetoxymethyl or amino group, the other substituent having the abovementioned meaning, and

X represents N-$R^5$, wherein $R^5$ represents hydrogen, a straight-chain-or branched alkyl radical which has up to 4 carbon atoms and is optionally interrupted by an oxygen atom, a morpholinoethyl radical, a phenyl radical or a benzyl radical, or

represents an oxygen atom, in which case one of the substituents $R^2$ or $R^3$ always denotes an amino group, by:

A) reacting ylidene-β-keto esters of the general formula (II)

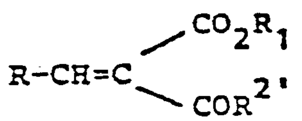

(II)

in which

R, $R^1$ and $R^{2'}$ have the abovementioned meaning, with enaminocarboxylic acid esters of the general formula (III)

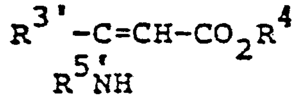

(III)

in which

$R^{3'}$, $R^5$ and $R^4$ have the abovementioned meaning, if appropriate in the presence of inert organic solvents, or

B) reacting ylidene-β-keto esters of the general formula (II)

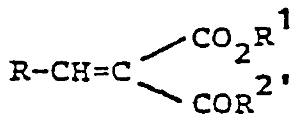

(II)

in which

R, $R^1$ and $R^{2'}$ have the abovementioned meaning, with amines of the general formula (IV) and β-ketocarboxylic acid esters of the general formula (V)

$R^5-NH_2$ (IV)

$R^{3'}-CO-CH_2-CO_2R^4$ (V)

in which
$R^5$, $R^{3'}$ and $R^4$ have the abovementioned meaning, if appropriate in the presence of inert organic solvents, or
C) reacting ylidene-β-keto esters of the general formula (VI)

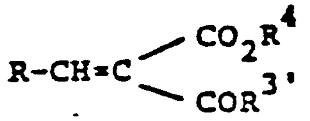

(VI)

in which
R, $R^{3'}$ and $R^4$ have the abovementioned meaning, with enaminocarboxylic acid esters of the general formula (VII)

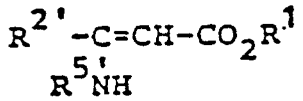

(VII)

in which
$R^{2'}$, $R^5$ and $R^1$ have the abovementioned meaning, if appropriate in the presence of inert organic solvents or
D) reacting ylidene-β-keto esters of the general formula (VI)

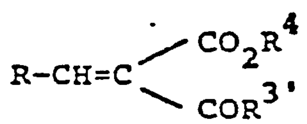

(VI)

in which
R, $R^{3'}$ and $R^4$ have the abovementioned meaning, with amines of the general formula (IV)
$R^5-NH_2$ (IV)
in which
$R^5$ has the abovementioned meaning, and β-ketocarboxylic acid esters of the general formula (VIII)
$R^{2'}-CO-CH_2-CO_2R^1$ (VIII)
in which
$R^{2'}$ and $R^1$ have the abovementioned meaning, if appropriate in the presence of inert organic solvents, or
E) reacting aldehydes of the general formula (IX)

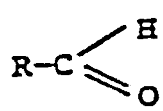

(IX)

in which
R has the abovementioned meaning, with enaminocarboxylic acid esters of the general formula (III)

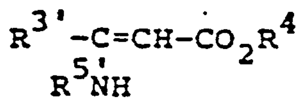 (III)

in which $R^{3'}$, $R^4$ and $R^5$ have the abovementioned meaning, and with β-ketocarboxylic acid esters of the general-formula (VIII)

$R^{2'}$-CO-$CH_2$-$CO_2R^1$ (VIII)

in which $R^1$ and $R^{2'}$ have the abovementioned meaning, if appropriate in the presence of inert organic solvents, or

F) reacting aldehydes of the general formula (IX)

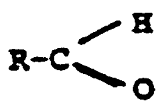 (IX)

in which R has the abovementioned meaning, with enaminocarboxylic acid esters of the general formula (VII)

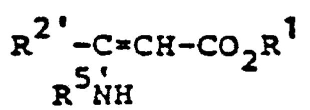 (VII)

in which $R^1$, $R^{2'}$ and $R^5$ have the abovementioned meaning and with β-ketocarboxylic acid esters of the general formula (V)

$R^{3'}$-CO-$CH_2$-$CO_2R^4$ (V)

in which $R^{3'}$ and $R^4$ have the abovementioned meaning, if appropriate in the presence of inert organic solvents, or

G) in the case where X in the formula (I) denotes NH and $R^2$ or $R^3$ represent an amino group, by reacting

1. ylidene-β-keto esters of the general formula (II)

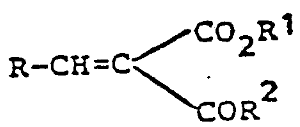 (II)

in which R, $R^1$ and $R^2$ have the abovementioned meaning but $R^2$ does not represent an amino or hydroxymethyl group, with amidinoacetic acid esters of the general formula (X)

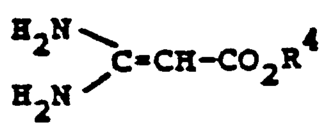 (X)

in which $R^4$ has the abovementioned meaning, if appropriate in the presence of inert organic solvents, or

2 ylidene-β-keto esters of the general formula (VI)

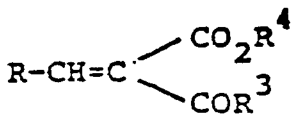

(VI)

in which

R, $R^3$ and $R^4$ have the abovementioned meaning, but $R^3$ does not represent an amino or hydroxylmethyl group, with amidinoacetic acid esters of the general formula (XI)

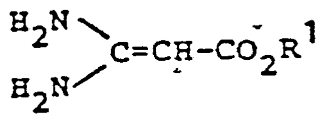

(XI)

in which

$R^1$ has the abovementioned meaning, if appropriate in the presence of inert organic solvents, or

H) in the case where X in the general formula (I) represents N-$R^5$, wherein $R^5$ has the abovementioned meaning and

R or $R^3$ represent the hydroxymethyl group, by subjecting the corresponding compounds according to the invention, in which $R^2$ or $R^3$ denote acetoxymethyl, to the conditions of an acid

or basic hydrolysis, the acetoxymethyl group being converted into a hydroxmethyl group

or

I) in the case where X in the general formula (I) represents

an oxygen atom and $R^2$ or $R^3$ represents an amino group, by

1. reacting ylidene-β-keto esters of the general formula (II)

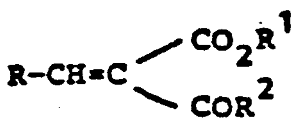

(II)

in which

R, $R^1$ and $R^2$ have the abovementioned meaning but $R^2$ does not represent the amino or hydroxymethyl group, with cyanoacetic acid esters of the general formula (XII)

$N\equiv C-CH_2CO_2R^4$ (XII)

in which

$R^4$ has the abovementioned meaning, if appropriate in the presence of organic solvents,

or

2. reacting ylidene-β-keto esters of the general formula (VI)

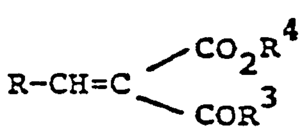

(VI)

in which R, $R^4$ and $R^3$ have the abovementioned meaning, but $R^3$ does not represent the amino or hydroxymethyl group, with cyanoacetic acid esters of the general formula (XIII)

$N\equiv C-CH_2-CO_2R^1$ (XIII)

in which

$R^1$ has the abovementioned meaning, if appropriate in the presence of organic solvents, and the compounds obtained of the general formula (I) are optionally converted into their addition salts.

7. Medicaments contain at least one compound of the general formula (I) according to Claim 1.

8. Process for the preparation of medicaments, characterised in that compounds of the general formula (I) according to Claim 1 are converted into a suitable form of administration' optionally using customary auxiliaries and excipients.

9. Medicaments with a long-lasting effect on the cardiovascular system, containing at least one compound of

the general formula (I) according to Claim 1.

10. Use of compounds of the general formula (I), according to Claim 1, for the preparation of hypotensive agents.

## Revendications

1. Composés de formule générale (I)

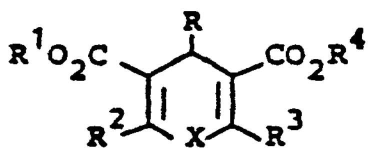

(I)

dans laquelle

R représente un groupe phényle, naphtyle ou thiényle, furyle, pyrryle, pyrazolyle, imidazolyle, oxazolyle, isoxazolyle, thiazolyle, pyridyle, pyridazinyle, pyrimidyle, pyrazinyle, indolyle, benzimidazolyle, benzoxazolyle, benzisoxazolyle, benzoxadiazolyle, quinolyle, isoquinolyle, quinazolyle ou quinoxalyle, les systèmes cycliques mentionnés pouvant être substitués chacun par un ou deux substituants identiques ou différents choisis dans le groupe comprenant le radical phényle, un radical alkyle à chaîne droite ou à chaîne ramifiée ayant 1 à 8 atomes de carbone, cycloalkyle ayant 3 à 7 atomes de carbone, alkoxy ayant 1 à 4 atomes de carbone, tri-, tétra- et pentaméthylène, dioxyméthylène, halogéno, trifluorométhyle, trifluorométhoxy, difluorométhoxy, tétrafluoréthoxy, nitro, cyano, azido ou $S0_m$-alkyle, où m désigne un nombre de 0 à 2 et le groupe alkyle contient de préférence 1 à 4 atomes de carbone,

$R^1$ désigne un reste d'hydrocarbure saturé ou insaturé à chaîne droite, ramifié ou cyclique ayant jusqu'à 20 atomes de carbone, qui est éventuellement interrompu dans la chaîne par 1 atome d'oxygène et/ou qui est éventuellement substitué par un groupe halogéno, cyano, hydroxy, acétoxy, phényle, phénoxy ou un groupe amino qui est substitué quant à lui par 2 substituants identiques ou différents choisis dans le groupe des radicaux alkyle ayant 1 à 4 atomes de carbone, phényle ou benzyle,

$R^4$ est un reste d'hydrocarbure aliphatique saturé ou insaturé à chaîne droite, ramifié ou cyclique ayant 7 à 14 atomes de carbone, qui est éventuellement interrompu dans la chaîne par un atome d'oxygène et/ou substitué le cas échéant par un halogène ou un radical hydroxy ou acétoxy,

$R^2$ et $R^3$ sont identiques ou différents et représentent chacun l'hydrogène, un reste alkyle à chaîne droite ou ramifié ayant jusqu'à 4 atomes de carbone, un reste phényle ou un reste benzyle, ou bien l'un des substituants $R^2$ ou $R^3$ représente un groupe hydroxyméthyle, acétoxyméthyle ou amino, l'autre substituant ayant la définition indiquée ci-dessus,

et

X est un groupe N-$R^5$, dans lequel $R^5$ représente l'hydrogène, un reste alkyle à chaîne droite ou ramifié, éventuellement interrompu par un atome d'oxygène, ayant jusqu'à 4 atomes de carbone, un reste morpholinoéthyle, un reste phényle ou un reste benzyle,

ou bien

un atome d'oxygène, l'un des substituants $R^2$ ou $R^3$ représentant alors toujours un groupe amino.

2. Composés de formule générale (I) suivant la revendication 1, dans lequel

R représente un groupe phényle, pyridyle, thiényle ou furyle, le noyau phényle étant alors éventuellement substitué par un ou deux substituants identiques ou différents du groupe des substituants phényle, alkyle ayant 1 à 4 atomes de carbone, alkoxy ayant 1 ou 2 atomes de carbone, fluor, chlore, brome, trifluorométhyle, trifluorométhoxy, difluorométhoxy, nitro, cyano, azido ou alkylmercapto ayant 1 à 4 atomes de carbone dans le reste alkyle,

$R^1$ désigne un reste d'hydrocarbure saturé ou insaturé à chaîne droite, ramifié ou cyclique, ayant jusqu'à 14 atomes de carbone, qui est éventuellement interrompu par un atome d'oxygène et/ou substitué le cas échéant par du fluor, du chlore, un radical cyano, hydroxy ou amino,

$R^4$ représente un reste d'hydrocarbure aliphatique saturé ou insaturé à chaîne droite, ramifié ou cyclique ayant 7 à 12 atomes de carbone, qui est éventuellement interrompu dans la chaîne par un atome d'oxygène et/ou substitué le cas échéant par du fluor, du chlore, un radical hydroxy ou acétoxy,

$R^2$ et $R^3$ sont identiques ou différents et représentent chacun l'hydrogène, un groupe alkyle ayant 1 ou 2 atomes de carbone, phényle ou benzyle,

ou bien

l'un des substituants $R^2$ ou $R^3$ désigne un groupe hydroxyméthyle, acétoxyméthyle ou amino, l'autre substituant ayant la définition indiquée ci-dessus,

X représente un groupe N-$R^5$ dans lequel $R^5$ est l'hydrogène, un reste alkyle ayant jusqu'à 4 atomes de carbone

qui est éventuellement interrompu par un atome d'oxygène, un reste morpholino-alkyle ayant 1 à 4 atomes de carbone dans la partie alkyle, un reste phényle ou un reste benzyle,

ou bien

un atome d'oxygène, l'un des substituants $R^2$ ou $R^3$ représentant alors toujours un groupe amino.

3. Composés de formule générale (I) suivant la revendication 1, dans laquelle

R représente un reste phényle qui contient un ou deux substituants identiques ou différents choisis dans le groupe chloro, trifluorométhyle, nitro ou cyano, ou représente un reste benzoxadiazolyle,

$R^1$ est un reste d'hydrocarbure à chaîne droite ou ramifié ayant jusqu'à 7 atomes de carbone, qui est éventuellement interrompu dans la chaîne par un atome d'oxygène et/ou qui est substitué le cas échéant par du fluor ou du chlore ou un groupe amino qui peut être substitué lui-meme par deux groupes méthyle ou par un groupe méthyle et un groupe benzyle,

$R^4$ est un reste d'hydrocarbure aliphatique saturé ou insaturé à chaîne droite, ramifié ou cyclique ayant 7 à 12 atomes de carbone, qui est éventuellement interrompu dans la chaîne par un atome d'oxygène ou qui est substitué éventuellement par du fluor, du chlore ou un radical hydroxy,

$R^2$ et $R^3$ représentent chacun l'hydrogène ou un reste alkyle ayant 1 ou 2 atomes de carbone, ou bien l'un des substituants $R^2$ ou $R^3$ représente le groupe amino, l'autre substituant ayant la définition indiquée ci-dessus, et

X est un groupe N-$R^5$, dans lequel $R^5$ est un atome d'hydrogène ou un reste alkyle ayant jusqu'à 3 atomes de carbone, ou bien

représente un atome d'oxygène, l'un des substituants $R^2$ ou $R^3$ représentant toujours un groupe amino.

4. Composés de formule générale (I) suivant la revendication 1, dans laquelle

$R^4$ désigne un reste d'hydrocarbure aliphatique saturé ou insaturé à chaîne droite, ramifié ou cyclique ayant jusqu'à 10 atomes de carbone, qui est éventuellement interrompu dans la chaîne par un atome d'oxygène ou qui est éventuellement substitué par du fluor, du chlore ou un radical hydroxy, et

les autres substituants possèdent la définition indiquée dans la revendication 2.

5. Composés de formule générale (I) suivant la revendication 1, destinés à être utilisés pour combattre des maladies circulatoires.

6. Procédé de production de composés de formule générale (I)

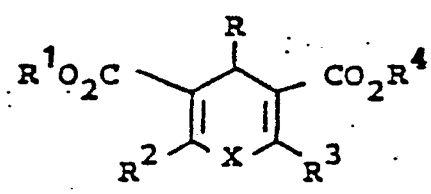

(I)

dans laquelle

R représente un groupe phényle, naphtyle ou thiényle, furyle, pyrryle, pyrazolyle, imidazolyle, oxazolyle, isoxazolyle, thiazolyle, pyridyle, pyridazinyle, pyrimidyle, pyrazinyle, indolyle, benzimidazolyle, benzoxazolyle, benzisoxazolyle, benzoxadiazolyle, quinolyle, isoquinolyle, quinazolyle ou quinoxalyle, les systèmes cycliques mentionnés pouvant être substitués chacun par un ou deux substituants identiques ou différents choisis dans le groupe comprenant le radical phényle, un radical alkyle à chaîne droite ou à chaîne ramifiée ayant 1 à 8 atomes de carbone, cycloalkyle ayant 3 à 7 atomes de carbone, alkoxy ayant 1 à 4 atomes de carbone, tri-, tétra- et pentaméthylène, dioxyméthylène, halogéno, trifluorométhyle, trifluorométhoxy, difluorométhoxy, tétrafluoréthoxy, nitro, cyano, azido ou $SO_m$-alkyle, où m désigne un nombre de 0 à 2 et le groupe alkyle contient de préférence 1 à 4 atomes de carbone,

$R^1$ désigne un reste d'hydrocarbure saturé ou insaturé à chaîne droite, ramifié ou cyclique ayant jusqu'à 20 atomes de carbone, qui est éventuellement interrompu dans la chaîne par 1 atome d'oxygène et/ou qui est éventuellement substitué par un groupe halogéno, cyano, hydroxy, acétoxy, phényle, phénoxy ou un groupe amino qui est substitué quant à lui par 2 substituants identiques ou différents choisis dans le groupe des radicaux alkyle ayant 1 à 4 atomes de carbone, phényle ou benzyle,

$R^4$ est un reste d'hydrocarbure aliphatique saturé ou insaturé à chaîne droite, ramifié ou cyclique ayant 7 à 14 atomes de carbone, qui est éventuellement interrompu dans la chaîne par un atome d'oxygène et/ou substitué le cas échéant par un halogène ou un radical hydroxy ou acétoxy,

$R^2$ et $R^3$ sont identiques ou différents et représentent chacun un hydrogène, un reste alkyle à chaîne droite ou ramifié ayant jusqu'à 4 atomes de carbone, un reste phényle ou un reste benzyle, ou bien l'un des substituants $R^2$ ou $R^3$ représente un groupe hydroxyméthyle, acétoxyméthyle ou amino, l'autre substituant ayant la définition indiquée ci-dessus,

et

X est un groupe N-$R^5$, dans lequel $R^5$ représente l'hydrogène, un reste alkyle à chaîne droite ou ramifié, éventuellement interrompu par un atome d'oxygène, ayant jusqu'à 4 atomes de carbone, un reste morpholinoéthyle, un reste phényle ou un reste benzyle,

ou bien

un atome d'oxygène, l'un des substituants $R^2$ ou $R^3$ représentant alors toujours un groupe amino,

dans lequel :

A)

On fait réagir des ylidène-β-cétoesters de formule générale (II)

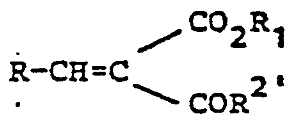

(II)

dans laquelle

R, $R^1$ et $R^{2'}$ ont la définition indiquée ci-dessus, avec des esters d'acides énaminocarboxyliques de formule générale (III)

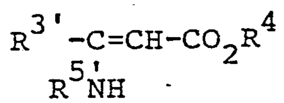

(III)

dans laquelle

$R^{3'}$, $R^5$ et $R^4$ ont la définition indiquée ci-dessus, éventuellement en présence de solvants organiques inertes, ou bien

B)

On fait réagir des ylidène-β-cétoesters de formule générale (II)

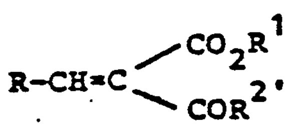

(II)

dans laquelle

R, $R^1$ et $R^2$ ont la définition indiquée ci-dessus, avec des amines de formule générale (IV) et des esters d'acide β-cétocarboxylique de formule générale (V)

$R^5$-$NH_2$ (IV)    $R^{3'}$-CO-$CH_2$-$CO_2R^4$ (V)

formules dans lesquelles

$R^5$, $R^{3'}$ et $R^4$ ont la définition indiquée ci-dessus, éventuellement en présence de solvants organiques inertes, ou bien

C)

on met en réaction des ylidène-β-cétoesters de formule générale (VI)

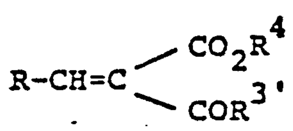

(VI)

dans laquelle

R, $R^{3'}$ et $R^4$ ont la définition indiquée ci-dessus, avec des esters d'acides énaminocarboxyliques de formule générale (VII)

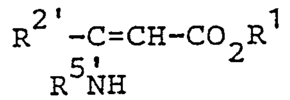

(VII)

dans laquelle $R^{2'}$, $R^5$ et $R^1$ ont la définition indiquée ci-dessus, éventuellement en présence de solvants organiques inertes, ou bien

D)

On fait réagir des ylidène-β-cétoesters de formule générale (VI)

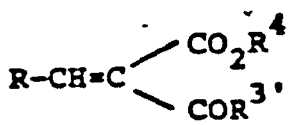

(VI)

dans laquelle

R, $R^{3'}$ et $R^4$ ont la définition indiquée ci-dessus, avec des amines de formule générale (IV)

$R^5$-$NH_2$ (IV)

dans laquelle

$R^5$ a la définition indiquée ci-dessus, et des esters d'acides β-cétocarboxyliques de formule générale (VIII)

$R^{2'}$-CO-$CH_2$-$CO_2R^1$ (VIII)

dans laquelle

$R^{2'}$ et $R^1$ ont la définition indiquée ci-dessus, éventuellement en présence de solvants organiques inertes ou bien

E)

On fait réagir des aldéhydes de formule générale (IX)

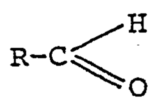

(IX)

dans laquelle

R a la définition indiquée ci-dessus, avec des esters d'acides énaminocarboxyliques de formule générale (III)

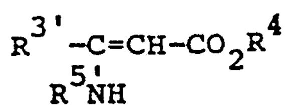

(III)

dans laquelle

$R^{3'}$, $R^4$ et $R^5$ ont la définition indiquée ci-dessus, et avec des esters d'acides β-cétocarboxyliques de formule générale (VIII)

$R^{2'}$-CO-$CH_2$-$CO_2R^1$ (VIII)

dans laquelle

$R^1$ et $R^{2'}$ ont la définition indiquée ci-dessus, éventuellement en présence de solvants organiques inertes, ou bien

F)

On met en réaction des aldéhydes de formule générale (IX)

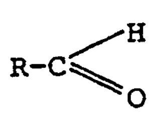

(IX)

dans laquelle

R a la définition indiquée ci-dessus, avec des esters d'acides énaminocarboxyliques de formule générale (VII)

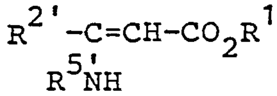

(VII)

dans laquelle

$R^1$, $R^{2'}$ et $R^5$ ont la définition indiquée ci-dessus et avec des esters d'acides β-cétocarboxyliques de formule générale (V)

$R^{3'}$-CO-$CH_2$-$CO_2R^4$ (V)

dans laquelle

$R^{3'}$ et $R^4$ ont la définition indiquée ci-dessus, éventuellement en présence de solvants organiques inertes ou bien

G)

au cas où X dans la formule (I) désigne NH et $R^2$ ou $R^3$

représente un groupe amino,

1. on fait réagir des ylidène-β-cétoesters de formule générale (II)

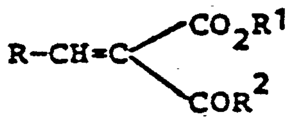

(II)

dans laquelle

R, $R^1$ et $R^2$ ont la définition indiquée ci-dessus, mais $R^2$ ne représente pas un groupe amino ou hydroxyméthyle, avec des esters d'acide amidino-acétique de formule générale (X)

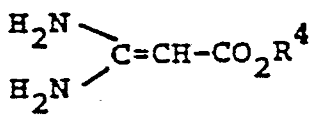

(X)

dans laquelle

$R^4$ a la définition indiquée ci-dessus, éventuellement en présence de solvants organiques inertes ou bien

2. on met en réaction des ylidène-β-cétoesters de formule générale (VI)

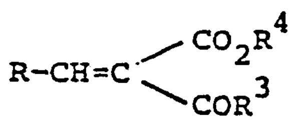

(VI)

dans laquelle

R, $R^3$ et $R^4$ ont la définition indiquée ci-dessus, mais $R^3$ ne représente pas un groupe amino ou hydroxyméthyle, avec des esters d'acide amidinoacétique de formule générale (XI)

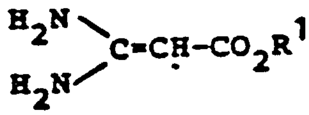

(XI)

dans laquelle

$R^1$ a la définition indiquée ci-dessus, éventuellement en présence de solvants organiques inertes, ou bien

H)

au cas où X dans la formule générale (I) représente N-$R^5$, où $R^5$ a la définition indiquée ci-dessus, et

R ou $R^3$ représente le groupe hydroxyméthyle, on soumet aux conditions d'une hydrolyse acide ou basique les

composés correspondants conformes à l'invention dans lesquels $R^2$ ou $R^3$ représente un groupe acétoxyméthyle, pour transformer le groupe acétoxyméthyle en un groupe hydroxyméthyle, ou bien

I)

au cas où X dans la formule générale (I) représente un atome d'oxygène et $R^2$ ou $R^3$ représente un groupe amino,

1. on fait réagir des ylidène-β-cétoesters de formule générale (II)

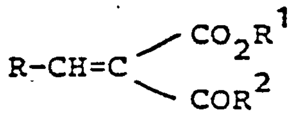

(II)

dans laquelle

R, $R^1$ et $R^2$ ont la définition indiquée ci-dessus, mais $R^2$ ne représente pas le groupe amino ou hydroxyméthyle, avec des esters d'acide cyanacétique de formule générale (XII)

$N{\equiv}C\text{-}C\text{-}CH_2\text{-}CO_2R^4$ (XII)

dans laquelle

$R^4$ a la définition indiquée ci-dessus,

éventuellement en présence de solvants organiques, ou bien

2. on met en réaction des ylidène-β-cétoesters de formule générale (VI)

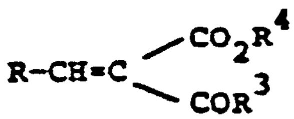

(VI)

dans laquelle

R, $R^4$ et $R^3$ ont la définition indiquée ci-dessus, mais $R^3$ ne représente pas le groupe amino ou hydroxyméthyle, avec des esters d'acide cyanacétique de formule générale (XIII)

$N{\equiv}C\text{-}CH_2\text{-}CO_2R^1$ (XIII)

dans laquelle

$R^1$ a la définition indiquée ci-dessus,

eventuellement en presence de solvants organiques, et on transforme éventuellement les composés obtenus de formule générale (I) en leurs sels d'addition.

7. Médicament contenant au moins un composé de formule générale (I) suivant la revendication 1.

8. Procédé de préparation de médicaments, caractérisé en ce qu'on convertit en une forme d'administration appropriée des composés de formule générale (I) suivant la revendication 1, en utilisant éventuellement des adjuvants et supports classiques.

9. Médicament à longue durée d'action sur le système circulatoire, contenant au moins un composé de formule générale (I) suivant la revendication 1.

10. Utilisation de composés de formule générale (I), suivant la revendication 1, pour la préparation de compositionsabaissant la pression sanguine.